# EUROPEAN PATENT SPECIFICATION

(11) **EP 4 453 943 B1**
(45) Date of publication and mention of the grant of the patent: **29.10.2025**
(21) Application number: 22794046.7
(22) Date of filing: 30.09.2022
(51) Int. Cl.: G16B 20/20, G06F 9/28, G06F 9/46, G06F 9/48, G06F 9/50, G06F 9/54, G16B 50/30, H04L 67/1008

(54) **FACILITATING SECURE EXECUTION OF EXTERNAL WORKFLOWS FOR GENOMIC SEQUENCING DIAGNOSTICS**
SICHERE AUSFÜHRUNG EXTERNER ARBEITSABLÄUFE FÜR GENOMISCHE SEQUENZIERUNGSDIAGNOSEN
FACILITATION DE L'EXÉCUTION SÉCURISÉE DE FLUX DE TRAVAIL EXTERNES POUR DES DIAGNOSTICS DE SÉQUENÇAGE GÉNOMIQUE

(30) Priority: 23.12.2021 US 202163293587 P; 26.09.2022 US 202217935476
(43) Date of publication of application: 30.10.2024
(73) Proprietor: ILLUMINA, INC., San Diego, CA 92122 (US)
(72) Inventor: SMOOT, Michael, San Diego, California 92122 (US); WARD, Jeremy, San Diego, California 92122 (US); FRENCH, Adam, Cambridge Cambridgeshire CB21 6DF (GB); TAYLOR, Russell, Cambridge Cambridgeshire CB21 6DF (GB)
(74) Representative: Robinson, David Edward Ashdown
(86) International application number: PCT/US2022/077405
(87) International publication number: WO 2023/122362

(56) References cited:
- EP-B1- 3 292 220
- CN-A- 112 748 997
- US-A1- 2021 158 939
- SCILIFELAB: "Enabling reproducible in silico data analyses with Nextflow - Paolo Di Tommaso", 5 October 2018 (2018-10-05), XP93071248, Retrieved from the Internet <URL:https://www.youtube.com/watch?v=jsxTC8pNPUc> [retrieved on 20230807]
- ANONYMOUS: "Kubernetes - Wikipedia", 8 December 2021 (2021-12-08), XP93071407, Retrieved from the Internet <URL:https://en.wikipedia.org/w/index.php?title=Kubernetes%26oldid=1059320560> [retrieved on 20230807]
- CHRISTIAN KNIEP: "Nextflow Introduction at the ISC Linux Container Workshop", 23 June 2017 (2017-06-23), XP093010733, Retrieved from the Internet <URL:https://www.youtube.com/watch?v=bN2tz0dn-Yg> [retrieved on 20221223]
- ANONYMOUS: "ICGC Argo Documentation - Analysis Pipelines Overview", 2 December 2021 (2021-12-02), XP093010756, Retrieved from the Internet <URL:https://docs.icgc-argo.org/docs/analysis-workflows/analysis-overview> [retrieved on 20221223]
- SHAN CHENGGANG ET AL: "Containerized Workflow Builder for Kubernetes", 2021 IEEE 23RD INT CONF ON HIGH PERFORMANCE COMPUTING & COMMUNICATIONS; 7TH INT CONF ON DATA SCIENCE & SYSTEMS; 19TH INT CONF ON SMART CITY; 7TH INT CONF ON DEPENDABILITY IN SENSOR, CLOUD & BIG DATA SYSTEMS & APPLICATION (HPCC/DSS/SMARTCITY/DEPENDSYS, 20 December 2021 (2021-12-20), pages 685 - 692, XP034129097, DOI: 10.1109/HPCC-DSS-SMARTCITY-DEPENDSYS53884.2021.00113

## Description

### BACKGROUND

In recent years, biotechnology firms and computer science institutions have improved hardware and software for generating diagnostics for nucleotide sequences of genomic samples. In particular, some existing diagnostic platforms generate nucleotide base calls from nucleotide reads of a sample nucleotide sequence and/or run diagnostics on nucleotide base calls for a variety of purposes. For example, an existing diagnostic system performs a diagnostic application to screen a nucleotide sequence for cancer (e.g., a cancer screening assay) by detecting specific genetic markers within nucleotide base calls of the sample sequence. Some existing diagnostic systems perform other diagnostics as well, such as genetic testing for other genetic conditions (or propensities for developing genetic conditions) or for determining other genetic traits.

Despite these recent advances, existing diagnostic systems continue to exhibit a number of drawbacks or disadvantages. For example, many conventional diagnostic systems are rigidly fixed to a specific set of internal diagnostic applications that are limited in their scope and utility. Indeed, many conventional systems can only perform genomic diagnostics using applications designed specifically for, and installed on, the system. Thus, in cases where a bioinformatician requires a particular diagnostic analysis of a nucleotide sequence, existing systems may be unable to provide the requisite analysis data if a platform-specific diagnostic application for the analysis has not yet been written and/or installed within the system.

Apart from being inflexible, some conventional diagnostic systems exhibit coding or network vulnerabilities that compromise or expose private or sensitive genetic data. To elaborate, for those existing systems that attempt to facilitate integration of external diagnostic applications, these systems often compromise the security of sequencing data (and other information) in exchange for the flexibility of allowing external workflows (including internal applications and/or external applications) to access the sequencing data to perform diagnostics. Indeed, some conventional systems are vulnerable to harmful external diagnostic workflows that either maliciously or inadvertently damage or corrupt sequencing data pertaining to nucleotide base calls and/or sequencing data relevant to other diagnostic applications. Consequently, many of these existing diagnostic systems cannot satisfy one or more diagnostic standards required by various regulatory bodies (e.g., in vitro diagnostic standards set by the United States Food and Drug Administration).

Additionally, some conventional diagnostic systems are inefficient. More specifically, existing systems often consume computing resources inefficiently when executing genomic diagnostic workflows. For example, some existing systems include no internal programming or other consideration for computing resource management, instead inundating available processors and memory with large numbers of (simultaneous) requests and instructions for the many processes involved in generating diagnostic data (often resulting in backlogs and slowdowns). Such existing systems are, therefore, either slow to generate requested diagnostic results for a diagnostic workflow or else fail to generate the results altogether, instead producing computational errors.

A container-native workflow engine for Kubernetes, which is suitable for diagnostic workflow, is known from the Argo Project, https://blog.argoproj.io/introducing-argo-a-container-native-workflow-engine-for-kubernetes-55c0b4b76fac.

Another pipeline orchestration tool for containerized genomic workflows, Nextflow, is known in the art for tackling problems of installation, deployment and maintenance of bioinformatic pipelines. Nextflow is a computational environment which provides a domain specific language (DSL), meant to simplify the writing of complex distributed computational pipelines in a portable and replicable manner. It allows the seamless parallelisation and deployment of any existing application with minimal development and maintenance overhead, irrespective of the original programming language, presented for instance at the ISC Linux Container Workshop 2017, https://www.youtube.com/watch?v=bN2tz0dn-Yg , and further introduction videos, such as "Enabling reproducible in silico data analyses with Nextflow" by Paolo Di Tommaso , SciLifeLab Big Talks, https://www.youtube.com/watch?v=jsxTC8pNPUc.

### SUMMARY

The invention is defined by the appended claims.

This disclosure describes embodiments of methods, non-transitory computer readable media, and systems that can flexibly, securely, and efficiently facilitate execution of external workflows for diagnostic analysis of nucleotide sequencing data, using Field-Programmable Gate Arrays (FPGAs) for processing diagnostic workflow containers. For example, the disclosed systems can utilize a container orchestration engine to allow external systems (e.g., third-party systems) to generate and implement workflows for analyzing sequencing data (e.g., sequencing data generated by a sequencing device and/or a variant analysis model). In some cases, the disclosed systems generate sequencing data, such as nucleotide base calls, and further utilize a container orchestration engine to implement diagnostic workflows for analyzing the sequencing data. For example, the disclosed systems can utilize the container orchestration engine to identify workflow containers that define individual functions of a workflow in a piece-wise fashion. In some such cases, the disclosed systems can request that the container orchestration engine implement an external sequencing diagnostic workflow-outside of workflows for a variant analysis model-and execute the external sequencing diagnostic workflow in a workflow container, executed on FPGA resources. In executing the individual workflow containers, the disclosed systems can also isolate the workflow containers to prevent access to, or corruption of, sequencing data or other workflow data.

### BRIEF DESCRIPTION OF THE DRAWINGS

The detailed description refers to the drawings briefly described below.
FIG. 1 illustrates a block diagram of a sequencing system including a diagnostic workflow system in accordance with one or more embodiments.
FIG. 2 illustrates an overview of executing an external sequencing diagnostic workflow in accordance with one or more embodiments.
FIG. 3 illustrates an example flow for executing an external sequencing diagnostic workflow on sequencing data utilizing a container orchestration engine in accordance with one or more embodiments.
FIG. 4 illustrates an example depiction of security permissions the diagnostic workflow system assigns to workflow containers of an external sequencing diagnostic workflow in accordance with one or more embodiments.
FIG. 5 illustrates utilizing a container orchestration engine to schedule execution of workflow containers in accordance with one or more embodiments.
FIG. 6 illustrates an example flow for determining whether diagnostic applications are compatible with an execution mode of a container orchestration engine in accordance with one or more embodiments.
FIG. 7 illustrates an example architecture diagram of the diagnostic workflow system in relation to an overall sequencing environment in accordance with one or more embodiments.
FIG. 8 illustrates a flowchart of a series of acts for executing an external sequencing diagnostic workflow in accordance with one or more embodiments.
FIG. 9 illustrates a block diagram of an example computing device for implementing one or more embodiments of the present disclosure.
FIG. 10 illustrates a block diagram of an example optical system for image-based genomic sequencing in accordance with one or more embodiments.
FIG. 11 illustrates an example imager for image-based genomic sequencing in accordance with one or more embodiments.
FIG. 12 illustrates an example diagram for performing image-based genomic sequencing in accordance with one or more embodiments.

### DETAILED DESCRIPTION

This disclosure describes embodiments of a diagnostic workflow system that facilitates execution of external workflows for diagnostic analysis of nucleotide sequencing data. In particular, the diagnostic workflow system can identify or generate sequencing data, such as nucleotide reads and nucleotide base calls for a sample nucleotide sequence. In addition, the diagnostic workflow system can perform different diagnostics on the sequencing data, either from internal workflows or from external workflows (that include sequencing diagnostic applications) to analyze the sequencing data. In some cases, the diagnostic workflow system implements a diagnostic workflow designed to analyze sequencing data to identify genetic markers for a specific disease or for hereditary traits of a sample. In certain embodiments, the diagnostic workflow is requested and/or implemented internally (e.g., not from a third-party device external to a server of the diagnostic workflow system). In other embodiments, the diagnostic workflow system requests to implement an external sequencing diagnostic workflow that includes applications compatible the diagnostic workflow system and/or applications sourced from outside the native variant analysis model (e.g., from the external system or another system). In implementing an external sequencing diagnostic workflow, the diagnostic workflow system can nevertheless maintain data security and integrity by utilizing a container orchestration engine to isolate or silo individual workflow containers for executing individual functions of the workflow without exposing sequencing data.

As just mentioned, in certain implementations, the diagnostic workflow system requests to perform a diagnostic analysis on a genomic sequence using an external sequencing diagnostic workflow. For instance, the diagnostic workflow system requests that a server implements an external sequencing diagnostic workflow that is not part of a suite of internal workflows for a variant analysis model and that is developed by an external entity. As a further example, in some cases, the diagnostic workflow system receives a request from a client device associated with an external system to process sequencing data to perform a diagnostic analysis (e.g., for a disease or a genetic condition). To facilitate external system access to internally generated sequencing data, the diagnostic workflow system can utilize a container orchestration engine designed to isolate and implement individual workflow containers. Specifically, the diagnostic workflow system can apply a container orchestration engine that allows a server or other computing device to access services and sequencing data of the diagnostic workflow system for tasks of the external sequencing diagnostic workflow-with granular control of how sequencing data is utilized in each individual process.

As mentioned, the diagnostic workflow system can utilize a container orchestration engine housed on a server device that runs a variant analysis model and that is located on a local network together with a genomic sequence processing device. Within the closed environment of the server device that includes the container orchestration engine and/or variant analysis model-and a local connection to the genomic sequence processing device -the diagnostic workflow system can securely perform sequencing diagnostics and enforce analysis protocols, even allowing external applications to access diagnostics results without risk of corruption or exposure of sequencing data. For example, the diagnostic workflow system can provide access permissions to sequencing data (or other data) on a specifically tailored basis for individual workflow containers (in relation to different types of data) to perform diagnostics-including for external sequencing diagnostic workflows from an external application. Depending on the execution mode of the container orchestration engine, the diagnostic workflow system can identify compatible diagnostic applications that satisfy security and analysis standards set by regulatory bodies (e.g., standards set by the United States Food and Drug Administration or some other body) for in vitro diagnostics (IVD). The diagnostic workflow system can likewise identify compatible diagnostic applications for investigational use only (IUO) analysis and research use only (RUO) analysis.

In some embodiments, the diagnostic workflow system schedules or allocates computing resources to execute a diagnostic workflow, such as an external sequencing diagnostic workflow. For example, the diagnostic workflow system utilizes the container orchestration engine to allocate computing resources, such as processing power and memory, to perform processes of individual workflow containers. To elaborate, the diagnostic workflow system can communicate with a genomic sequence processing device, such as a field programmable gate array (FPGA) or a central processing unit (CPU), designed and programmed to execute functions for diagnostic workflows. In some cases, the genomic sequence processing device can execute a limited number (e.g., one) of processes at a time. Consequently, the diagnostic workflow system utilizes resource allocation capabilities of the container orchestration engine to schedule execution of workflow containers of an external sequencing diagnostic workflow (e.g., to execute one after the other as processes are completed for each container) according to available computing resources.

As suggested above, embodiments of the diagnostic workflow system provide several advantages, benefits, and/or improvements over existing diagnostic systems. For instance, in some embodiments, the diagnostic workflow system is more flexible than many existing diagnostic systems. While many existing systems are limited to implementing system-specific, internal diagnostic applications and workflows to perform diagnostics for genomic sequencing data, the diagnostic workflow system can facilitate execution of external (e.g., third-party) sequencing diagnostic applications and workflows. Specifically, the diagnostic workflow system can utilize a container orchestration engine to enable receipt of an external sequencing diagnostic workflow (e.g., via upload) and to execute the external sequencing diagnostic workflow to perform a diagnostic analysis on nucleotide base calls (or other sequencing data) of a sample nucleotide sequence (as dictated by an external, not locally networked system). In some embodiments, the diagnostic workflow system can even (unlike prior systems) flexibly execute an external sequencing diagnostic workflow during (or after) a sequencing process for generating nucleotide base calls of a sample nucleotide sequence.

In addition to increased flexibility, in some embodiments, the diagnostic workflow system improves data security compared to conventional diagnostic systems. While some prior systems may attempt to facilitate diagnostic analysis from external applications, these conventional systems risk exposure and corruption of sequencing data or code for diagnostic applications when implementing workflows outside (or not native to) a variant analysis model or other internal application used for diagnostics. Indeed, existing diagnostic systems lack a mechanism or model for securely running third-party or other external applications on a computing device that runs a software application configured for genetic diagnostics.

By contrast, the diagnostic workflow system maintains security and integrity of sequencing data even when implementing external sequencing diagnostic workflows from sources outside of a secure local network of the diagnostic workflow system. Unlike existing diagnostic systems that may expose genetic information for a sample, for example, the diagnostic workflow system utilizes a container orchestration engine that isolates individual workflow containers defining processes of an external sequencing diagnostic workflow, preventing unwanted or even accidental exposure to sensitive sequencing data or other genetic information. By executing a workflow container for an external sequencing diagnostic workflow by itself-and configuring security permissions targeted for the external sequencing diagnostic workflow-the diagnostic workflow system encodes a secure execution of the external sequencing diagnostic workflow without access to internal metrics for sequencing run software or a variant analysis model. Indeed, unlike existing diagnostics systems, the diagnostic workflow system goes beyond standard encryption by securing external sequencing diagnostic workflows in separate containers and granting targeted security permissions (e.g., read-only permissions) to such containers to prevent the external sequencing diagnostic workflows from corrupting a code or internal metrics of applications for sequencing runs or variant analysis. As a result of its improved security and analysis protocols, the diagnostic workflow system can (unlike some prior systems) satisfy various standards for IVD.

Further, in certain embodiments, the diagnostic workflow system improves computational efficiency over existing diagnostic systems by orchestrating workflows to execute using limited resources of an FPGA or CPU. For instance, rather than inundating available processors and other computing resources with excessive processing requests for a diagnostic workflow like some existing systems, the diagnostic workflow system can utilize a container orchestration engine to intelligently allocate computing resources for executing individual workflow containers. Specifically, the diagnostic workflow system can utilize the container orchestration engine to designate iterative or sequential processing of respective workflow containers (coordinated together with or after processing of sequencing data) via a genomic sequence processing device, such as an FPGA or a CPU. The diagnostic workflow system can thus prevent backlogs and/or slowdowns prevalent in prior systems when executing diagnostic workflows on sequencing data.

As suggested by the foregoing discussion, this disclosure utilizes a variety of terms to describe features and benefits of the diagnostic workflow system. Additional detail is hereafter provided regarding the meaning of these terms as used in this disclosure. As used in this disclosure, for instance, the term "sample nucleotide sequence" or "sample sequence" refers to a sequence of nucleotides isolated or extracted from a sample organism (or a copy of such an isolated or extracted sequence). In particular, a sample nucleotide sequence includes a segment of a nucleic acid polymer that is isolated or extracted from a sample organism and composed of nitrogenous heterocyclic bases. For example, a sample nucleotide sequence can include a segment of deoxyribonucleic acid (DNA), ribonucleic acid (RNA), or other polymeric forms of nucleic acids or chimeric or hybrid forms of nucleic acids noted below. More specifically, in some cases, the sample nucleotide sequence is found in a sample prepared or isolated by a kit and received by a sequencing device.

As further used herein, the term "sequencing data" refers to data or information pertaining to a nucleotide sequence for one or more genomic samples. For example, sequencing data can include data generated by a sequencing device and/or a variant analysis model. In some cases, sequencing data includes nucleotide reads, nucleotide base calls, and/or sequencing metrics associated with a sample nucleotide sequence. In one or more embodiments, sequencing data is specific to a particular nucleotide sequence and is generated using proprietary methods and processes of a genomic analysis platform that includes a variant analysis model implemented by a genomic sequence processing device.

In certain embodiments, sequencing data is stored or located on one or more workflow data sources. The term "workflow data source" refers to a network storage location or repository for storing one or more types of sequencing data. For example, a workflow data source can include a location specific to a certain type of sequencing data such as a input directory, an output directory, or an application directory. Within an input directory, sequencing data can include data from a sample nucleotide sequence written or generated by a variant analysis model (e.g., nucleotide base calls). Within an output directory, sequencing data can include output information written or generated by a container orchestration engine performing a workflow (e.g., diagnostics results). Within an application directory, sequencing data can include a definition of a diagnostic application and/or a diagnostic workflow (e.g., an external sequencing diagnostic workflow), including indications for input, outputs, and other execution information for performing diagnostics.

Relatedly, the term "nucleotide base call" (or sometimes simply "call") refers to a determination or prediction of a particular nucleotide base (or nucleotide base pair) for a genomic coordinate of a sample genome or for an oligonucleotide during a sequencing cycle. In particular, a nucleotide base call can indicate (i) a determination or prediction of the type of nucleotide base that has been incorporated within an oligonucleotide on a nucleotide-sample slide (e.g., read-based nucleotide base calls) or (ii) a determination or prediction of the type of nucleotide base that is present at a genomic coordinate or region within a sample genome, including a variant call or a non-variant call in a digital output file. In some cases, for a nucleotide read, a nucleotide base call includes a determination or a prediction of a nucleotide base based on intensity values resulting from fluorescent-tagged nucleotides added to an oligonucleotide of a nucleotide-sample slide (e.g., in a well of a flow cell). Alternatively, a nucleotide base call includes a determination or a prediction of a nucleotide base to chromatogram peaks or electrical current changes resulting from nucleotides passing through a nanopore of a nucleotide-sample slide. By contrast, a nucleotide base call can also include an initial or final prediction of a nucleotide base at a genomic coordinate of a sample genome for a variant call file or other base call output file-based on nucleotide reads corresponding to the genomic coordinate. Accordingly, a nucleotide base call can include a base call corresponding to a genomic coordinate and a reference genome, such as an indication of a variant or a non-variant at a particular location corresponding to the reference genome. Indeed, a nucleotide base call can refer to a variant call, including but not limited to, a single nucleotide polymorphism (SNP), an insertion or a deletion (indel), or base call that is part of a structural variant. By using nucleotide base call, a sequencing system determines a sequence of a nucleic acid polymer. For example, a single nucleotide base call can comprise an adenine call, a cytosine call, a guanine call, or a thymine call for DNA (abbreviated as A, C, G, T) or a uracil call (instead of a thymine call) for RNA (abbreviated as U).

As used herein, the term "sequencing metric" refers to a quantitative measurement or score indicating a degree to which an individual nucleotide base call (or a sequence of nucleotide base calls) aligns, compares, or quantifies with respect to a genomic coordinate or genomic region of a reference genome, with respect to nucleotide base calls from nucleotide reads, or with respect to external genomic sequencing or genomic structure. For instance, a sequencing metric includes a quantitative measurement or score indicating a degree to which (i) individual nucleotide base calls align, map, or cover a genomic coordinate or reference base of a reference genome; (ii) nucleotide base calls compare to reference or alternative nucleotide reads in terms of mapping, mismatch, base call quality, or other raw sequencing metrics; or (iii) genomic coordinates or regions corresponding to nucleotide base calls demonstrate mappability, repetitive base call content, DNA structure, or other generalized metrics.

Relatedly, as used herein, the term "nucleotide read" (or sometimes simply "read") refers to an inferred sequence of one or more nucleotide bases (or nucleotide base pairs) from all or part of a sample nucleotide sequence. In particular, a nucleotide read includes a determined or predicted sequence of nucleotide base calls for a nucleotide fragment (or group of monoclonal nucleotide fragments) from a sequencing library corresponding to a genome sample. For example, the diagnostic workflow system determines a nucleotide read by generating nucleotide base calls for nucleotide bases passed through a nanopore of a nucleotide-sample slide, determined via fluorescent tagging, or determined from a well in a flow cell.

As noted, in some embodiments, the diagnostic workflow system utilizes a variant analysis model to generate a nucleotide base call for a genomic coordinate. As used herein, the term "variant analysis model" refers to a model including an algorithm or a set of algorithms for analyzing data for a sample nucleotide sequence (e.g., base call data). In some cases, a variant analysis model is a probabilistic model that generates sequencing data from nucleotide reads of a sample nucleotide sequence, including nucleotide base calls (e.g., variant calls) and associated metrics (e.g., base call quality metrics). For example, in some cases, a variant analysis model refers to a Bayesian probability model that generates variant calls based on nucleotide reads of a sample nucleotide sequence. Such a model can include a model for secondary analysis performed by a server executing variant-call software to align samples' nucleotide reads with a reference genome, determine genetic variants of samples based on the aligned nucleotide reads with respect to the reference genome, and determine one or more of quality metrics, allele frequency metrics, or other sequencing metrics. A variant analysis model may likewise include multiple components, including, but not limited to, different software applications or components for mapping and aligning, sorting, duplicate marking, computing read pileup depths, and variant calling. In some cases, the variant analysis model refers to the ILLUMINA DRAGEN model for variant calling functions and mapping and alignment functions.

As mentioned, in some embodiments, the diagnostic workflow system utilizes a container orchestration engine to orchestrate execution of an external sequencing diagnostic workflow. As used herein, the term "container orchestration engine" refers to an application for automating deployment, scaling, and management of containerized software services and applications. For example, a container orchestration engine can include a software application having a microservice architecture that executes individual workflow containers as part of a diagnostic workflow (e.g., an external sequencing diagnostic workflow). The container orchestration engine can treat each container separately for performing discrete functionalities (e.g., containerized tasks) that can be compartmentalized and added or removed from workflows in a piecewise fashion.

Relatedly, the term "workflow container" (or sometimes simply "container") refers to a unit of software that packages code (and all its dependencies) for portable deployment. For example, a workflow container includes a compartmentalized or containerized, manipulable, moveable, and executable body of code that performs a particular function or task. In some cases, a workflow container is executable to perform a function or task (e.g., a process or thread) to, for instance, generate a particular output from a piece of sequencing data (e.g., a final output or an intermediate output that feeds into another container). The diagnostic workflow system can treat workflow containers separately, isolating some containers differently than others to permit and/or prevent access to sequencing data in a specific and tailored fashion (e.g., within one or more workflow data sources). In some cases, a container refers to a NEXTFLOW container and/or a KUBERNETES container.

Additionally, as used herein, the term "sequencing diagnostic workflow" refers to a collection of tasks or functions organized and orchestrated together to product a set of diagnostic outputs from sequencing data for a sample nucleotide sequence. For example, a sequencing diagnostic workflow may comprise any number of tasks that provide any number of functions which may include secondary analysis, tertiary analysis, custom QC logic, reporting, or other desired functionality. In some cases, multiple entities can develop a single workflow that can be deployed locally (e.g., at an edge server) or in the cloud.

An "external sequencing diagnostic workflow" refers to a sequencing diagnostic workflow that is external to a genomic analysis software platform or external to one or more applications from a larger genomic analysis software platform. For instance, an external sequencing diagnostic workflow includes a sequencing diagnostic workflow that is not native to (or not integrated as part of) software for a variant analysis model (or another software application or set of software applications) that is used for diagnostics and in compliance with standardized genetic diagnostic protocols. In some cases, the diagnostic workflow system receives or identifies an external sequencing diagnostic workflow from a third-party system that does not share a local network with a server device hosting the diagnostic workflow system (e.g., with the container orchestration engine and/or the variant analysis model). An external sequencing diagnostic workflow can refer to a newly generated workflow or a modified version of a pre-existing workflow defined by an external device operated by an external entity (e.g., an entity not part of the diagnostic workflow system). In some cases, an external sequencing diagnostic workflow can refer to add-on software for the diagnostic workflow system that is compatible to plug in to a variant analysis model for performing secondary and/or tertiary analysis of nucleotide base calls.

An external sequencing diagnostic workflow can be part of, or defined by, a diagnostic application (e.g., an external diagnostic application). A "diagnostic application" can refer to a package of custom workflow content deployable by the diagnostic workflow system and that includes workflow definitions, containerized tasks, custom user interface, custom microservices, and/or reference data. A diagnostic application can have a particular structure or file type (e.g., tape archive or "TAR" files) that define workflows and other application data. In some embodiments, a diagnostic application is a single deployable unit that can be installed on a system that is completely disconnected from the Internet. An application package can be signed to ensure authenticity and validity. The package can be uploaded to a server via a UI portal (e.g., using a browser). In some cases, an external sequencing diagnostic workflow is part of an external diagnostic application generated by (and received from) an external (e.g., third party) system.

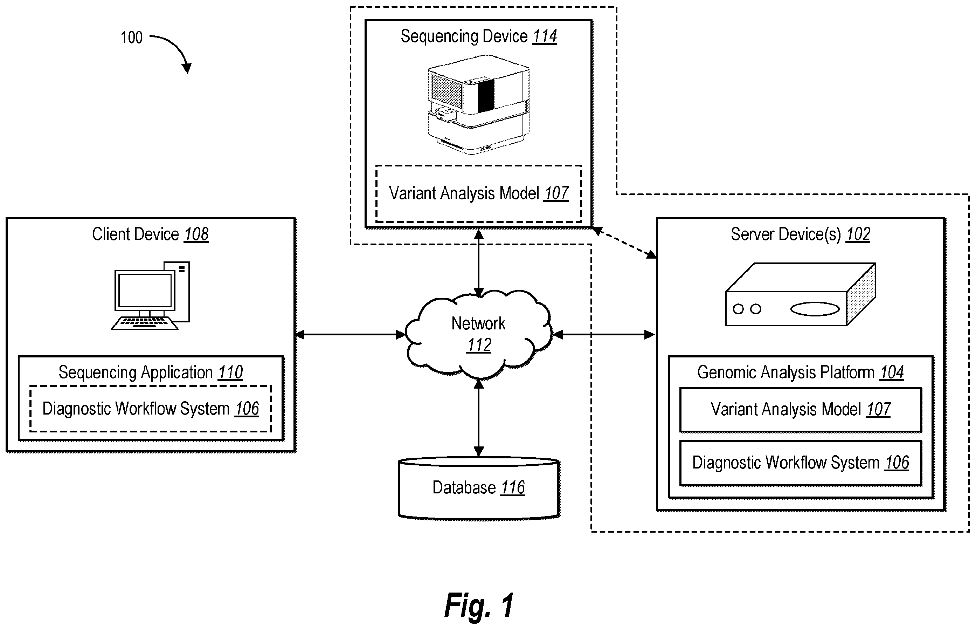

The following paragraphs describe the diagnostic workflow system with respect to illustrative figures that portray example embodiments and implementations. For example, FIG. 1 illustrates a schematic diagram of a system environment (or "environment") 100 in which a diagnostic workflow system 106 operates in accordance with one or more embodiments. As illustrated, the environment 100 includes one or more server device(s) 102 connected to a client device 108 and a sequencing device 114 via a network 112. While FIG. 1 shows an embodiment of the diagnostic workflow system 106, this disclosure describes alternative embodiments and configurations below.

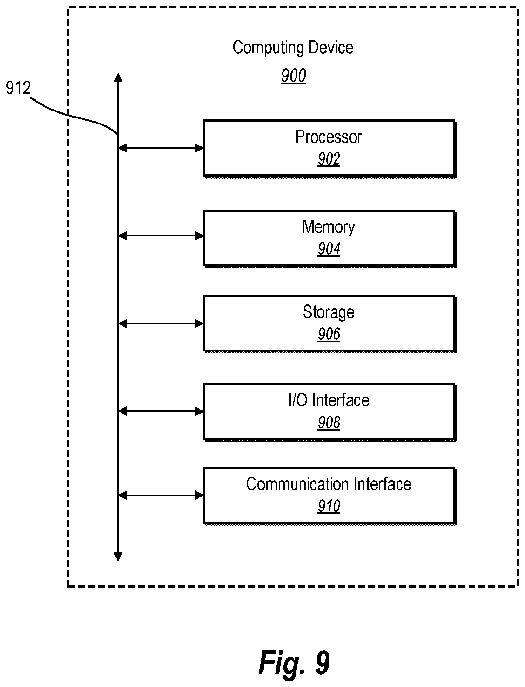

As shown in FIG. 1, the server device(s) 102, the client device 108, and the sequencing device 114 can communicate with each other via the network 112. The network 112 comprises any suitable network over which computing devices can communicate. Example networks are discussed in additional detail below with respect to FIG. 9.

As indicated by FIG. 1, the sequencing device 114 comprises a device for sequencing a nucleic acid polymer. In some embodiments, the sequencing device 114 analyzes nucleic acid segments or oligonucleotides extracted from samples to generate nucleotide reads or other data utilizing computer implemented methods and systems (described herein) either directly or indirectly on the sequencing device 114. More particularly, the sequencing device 114 receives and analyzes, within nucleotide-sample slides (e.g., flow cells), nucleic acid sequences extracted from samples. In one or more embodiments, the sequencing device 114 utilizes SBS to sequence nucleic acid polymers into nucleotide reads. In addition or in the alternative to communicating across the network 112, in some embodiments, the sequencing device 114 bypasses the network 112 and communicates directly with the server device(s) 102 (and/or the client device 108). Indeed, in some embodiments, the sequencing device 114 and the server device(s) 102 share a local network (e.g., housed on the same or different servers), as indicated by the dashed box, while the client device 108 does not share the local network and instead communicates via the network 112.

As further indicated by FIG. 1, the server device(s) 102 may generate, receive, analyze, store, and transmit digital data, such as data for determining nucleotide base calls, sequencing nucleic acid polymers, and/or performing diagnostics on a nucleotide sequence. As shown in FIG. 1, the sequencing device 114 may send (and the server device(s) 102 may receive) call data from the sequencing device 114. The server device(s) 102 may also communicate with the client device 108. In particular, the server device(s) 102 can send data to the client device 108, including a variant call file or other information indicating nucleotide base calls, sequencing metrics, error data, diagnostic information, or other metrics associated with a nucleotide base call.

In some embodiments, the serve device(s) 102 comprise a local server device that is located at or near a same physical location of the sequencing device 114. Indeed, in some embodiments, the serve device(s) 102 and the sequencing device 114 are integrated into a same computing device, as indicated by dotted lines around the server device(s) 102 and the sequencing device 114.

Rather than placed locally with the sequencing device 114, in some embodiments, the server device(s) 102 comprise a distributed collection of servers where the server device(s) 102 include a number of server devices distributed across the network 112 and located in the same or different physical locations. As suggested, in some cases, the server device(s) 102 house the genomic analysis platform 104. Further, the server device(s) 102 can comprise a content server, an application server, a communication server, a web-hosting server, or another type of server.

As further shown in FIG. 1, the server device(s) 102 can include a genomic analysis platform 104 for generating and analyzing sequencing data. Generally, the genomic analysis platform 104 includes a variant analysis model 107 that generates and/or analyzes call data, such as sequencing metrics received from the sequencing device 114, to determine nucleotide base sequences for nucleic acid polymers. For example, the variant analysis model 107 can receive raw data from the sequencing device 114 and can determine a nucleotide base sequence for a nucleic acid segment. In some embodiments, the variant analysis model 107 determines the sequences of nucleotide bases in DNA and/or RNA segments or oligonucleotides. In addition to processing and determining sequences for nucleic acid polymers, the variant analysis model 107 also generates a variant call file indicating one or more nucleotide base calls and/or variant calls for one or more genomic coordinates. Further, in some embodiments, the genomic analysis platform 104 includes the diagnostic workflow system 106.

As just mentioned, and as illustrated in FIG. 1, the diagnostic workflow system 106 analyzes sequencing data, such as call data and/or sequencing metrics (e.g., from the sequencing device 114 or the variant analysis model 107) to generate or determine various diagnostics. For example, the diagnostic workflow system 106 performs a diagnostic analysis of sequencing data for a sample nucleotide sequence. In some cases, the diagnostic workflow system 106 performs a diagnostic analysis to diagnose, or determine propensities for, one or more diseases or genetic conditions. Indeed, the diagnostic workflow system 106 executes an external sequencing diagnostic workflow (as received from the client device 108) to perform a diagnostic on sequencing data to determine a likelihood of manifesting a genetic condition or trait.

As further illustrated and indicated in FIG. 1, the client device 108 can generate, store, receive, and send digital data. In particular, the client device 108 can receive sequencing metrics from the sequencing device 114. Furthermore, the client device 108 may communicate with the server device(s) 102 to receive a variant call file comprising nucleotide base calls and/or other metrics, such as a call-quality, a genotype indication, and a genotype quality. The client device 108 can accordingly present or display information pertaining to the nucleotide base call within a graphical user interface to a user associated with the client device 108. In addition, the client device 108 can generate and provide (e.g., upload via the network 112) an external sequencing diagnostic workflow that includes one or more workflow containers for performing a diagnostic analysis of sequencing data. Indeed, the client device 108 can receive, via a graphical user interface, user interaction selecting and arranging or organizing workflow containers for an extemal sequencing diagnostic workflow. The client device 108 can also receive a diagnostic result from the server device(s) 102 and can display the diagnostic result within a graphical user interface.

The client device 108 illustrated in FIG. 1 may comprise various types of client devices. For example, in some embodiments, the client device 108 includes non-mobile devices, such as desktop computers or servers, or other types of client devices. In yet other embodiments, the client device 108 includes mobile devices, such as laptops, tablets, mobile telephones, or smartphones. Additional details regarding the client device 108 are discussed below with respect to FIG. 9.

As further illustrated in FIG. 1, the client device 108 includes a sequencing application 110. The sequencing application 110 may be a web application or a native application stored and executed on the client device 108 (e.g., a mobile application, desktop application). The sequencing application 110 can include instructions that (when executed) cause the client device 108 to receive data from the diagnostic workflow system 106 and present, for display at the client device 108, data from a variant call file. Furthermore, the sequencing application 110 can instruct the client device 108 to display a visualization of an external sequencing diagnostic workflow and/or workflow containers to arrange within the external sequencing diagnostic workflow, as well as a diagnostic result received from the server device(s) upon executing the external sequencing diagnostic workflow.

As further illustrated in FIG. 1, the diagnostic workflow system 106 may be located on the client device 108 as part of the sequencing application 110 or on the sequencing device 114. Accordingly, in some embodiments, the diagnostic workflow system 106 is implemented by (e.g., located entirely or in part) on the client device 108. In yet other embodiments, the diagnostic workflow system 106 is implemented by one or more other components of the environment 100, such as the sequencing device 114. In particular, the diagnostic workflow system 106 can be implemented in a variety of different ways across the server device(s) 102, the network 112, the client device 108, and the sequencing device 114. For example, the diagnostic workflow system 106 can be downloaded from the server device(s) 102 to the client device 108 and/or to the sequencing device 114 where all or part of the functionality of the diagnostic workflow system 106 is performed at each respective device within the environment 100.

As further illustrated in FIG. 1, the environment 100 includes a database 116. The database 116 can store information such as external sequencing diagnostic workflows, diagnostics results, variant call files, sample nucleotide sequences, and sequencing data such as nucleotide reads, nucleotide base calls, variant calls, and sequencing metrics. In some embodiments, the server device(s) 102, the client device 108, and/or the sequencing device 114 communicate with the database 116 (e.g., via the network 112) to store and/or access information, such as external sequencing diagnostic workflows, diagnostics results, variant call files, sample nucleotide sequences, and sequencing data such as nucleotide reads, nucleotide base calls, variant calls, and sequencing metrics. In some cases, the database 116 also stores one or more models, such as the variant analysis model 107.

Though FIG. 1 illustrates the components of environment 100 communicating via the network 112, in certain implementations, the components of environment 100 can also communicate directly with each other, bypassing the network 112. For instance, and as previously mentioned, in some implementations, the client device 108 communicates directly with the sequencing device 114. Additionally, in some embodiments, the client device 108 communicates directly with the diagnostic workflow system 106. Moreover, the diagnostic workflow system 106 can access one or more databases housed on or accessed by the server device(s) 102 or elsewhere in the environment 100.

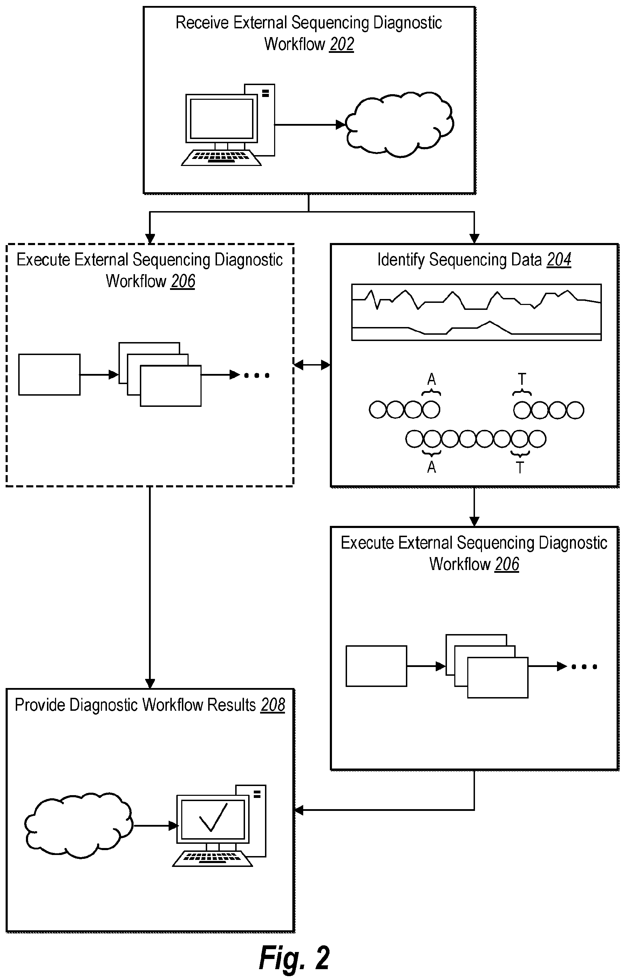

As mentioned, in certain described embodiments, the diagnostic workflow system 106 executes an external sequencing diagnostic workflow. In particular, the diagnostic workflow system 106 executes an external sequencing diagnostic workflow generated by an external system or an external device operated by an external system, such as a third-party system or a third-party device (e.g., the client device 108) to perform a diagnostic analysis of sequencing data. FIG. 2 illustrates an example overview of executing an external sequencing diagnostic workflow in accordance with one or more embodiments. FIG. 2 provides a general overview of executing an external sequencing diagnostic workflow, and additional detail regarding the various acts is provided thereafter with reference to subsequent figures.

As illustrated in FIG. 2, the diagnostic workflow system 106 performs an act 202 to receive an external sequencing diagnostic workflow. More specifically, the diagnostic workflow system 106 receives the external sequencing diagnostic workflow generated by an external system. In some embodiments, the diagnostic workflow system 106 receives the external sequencing diagnostic workflow directly from an external device such as the client device 108. In other embodiments, the diagnostic workflow system 106 receives the external sequencing diagnostic workflow from a different server (e.g., a web hosting server) that communicates with the client device 108 by hosting a website whereby the client device 108 uploads the external sequencing diagnostic workflow.

Indeed, the diagnostic workflow system 106 receives the external sequencing diagnostic workflow from a web hosting server that receives the external sequencing diagnostic workflow (as part of an application) as an upload. In some embodiments, the web hosting server is part of the diagnostic workflow system 106, while in other embodiments the web hosting server is external to the diagnostic workflow system 106. Thus, in some cases, the diagnostic workflow system 106 receives the external sequencing diagnostic workflow while in other cases the external sequencing diagnostic workflow is already downloaded or otherwise included as part of the diagnostic workflow system 106.

As further illustrated in FIG. 2, the diagnostic workflow system 106 performs an act 204 to identify sequencing data. To elaborate, the diagnostic workflow system 106 identifies sequencing data such as nucleotide base calls and/or sequencing metrics associated with one or more sample nucleotide sequences. In some cases, the diagnostic workflow system 106 generates the sequencing data utilizing a variant analysis model (e.g., the variant analysis model 107), while in other cases the diagnostic workflow system 106 receives the sequencing data from another server hosting the variant analysis model. In certain embodiments, the diagnostic workflow system 106 identifies sequencing data specific to a particular sample.

Additionally, the diagnostic workflow system 106 performs an act 206 to execute an external sequencing diagnostic workflow. Specifically, the diagnostic workflow system 106 executes the external sequencing diagnostic workflow by performing a set of workflow containers that make up the external sequencing diagnostic workflow. For instance, the diagnostic workflow system 106 executes the external sequencing diagnostic workflow generated by an external system and/or uploaded by an external device (e.g., the client device 108). In some cases, the diagnostic workflow system 106 receives the external sequencing diagnostic workflow from a server that directly receives the external sequencing diagnostic workflow via upload and relays the external sequencing diagnostic workflow to a server (e.g., the server device(s) 102) of the diagnostic workflow system 106.

Indeed, the diagnostic workflow system 106 executes an external sequencing diagnostic workflow that is generated by an external system operated by an external entity. To elaborate, the diagnostic workflow system 106 facilitates or enables generation and/or arrangement of an external sequencing diagnostic workflow via an external device (e.g., the client device 108). For instance, the external device can arrange or organize individual workflow containers for performing discretized functions or tasks as part of performing a particular diagnostic test on a sample nucleotide sequence. In some embodiments, the workflow containers are predefined or pre-generated by the diagnostic workflow system 106 and stored for use (by an external system) in an arrangement of an external sequencing diagnostic workflow. In other embodiments, the diagnostic workflow system 106 facilitates generation of entirely new workflow containers by an external system.

In some cases, the external sequencing diagnostic workflow is part of an external application. For example, the diagnostic workflow system 106 can execute an external sequencing diagnostic workflow that is defined by an application. The application can include custom user interfaces, reference data, and a workflow that includes various constituent containers, as defined by an external system. The diagnostic workflow system 106 can implement the external application to execute an external sequencing diagnostic workflow and can provide diagnostic results via a web user interface (e.g., a custom interface as defined by the application).

In certain embodiments, the external application is a modified version of an internal application pre-generated and stored within the diagnostic workflow system 106 (e.g., pre-authorized to comply with one or more standardized genetic diagnostic protocols such as IVD or to comply with other analysis protocols such as IUO or RUO). The diagnostic workflow system 106 can receive or identify a modified version of an internal application that is modified or generated by an external system. Indeed, an external system may modify an internal application to alter, add, and/or remove one or more workflow containers within a workflow. For instance, if a stored internal application includes a workflow that performs diagnostics on certain nucleotide base calls to identify a particular genetic condition (e.g., a TSO500 screening assay designed to detect certain cancer markers), the external system can modify the application (thereby generating an external application) to analyze different nucleotide base calls and/or to process other sequencing data in a slightly different way.

To execute the external sequencing diagnostic workflow, the diagnostic workflow system 106 requests that a container orchestration engine implement the external sequencing diagnostic workflow for performing a diagnostic analysis on nucleotide base calls for a sample nucleotide sequence. More specifically, the diagnostic workflow system 106 requests that a container orchestration engine implement the external sequencing diagnostic workflow according to its arrangement as defined in an external diagnostic application. For instance, the diagnostic workflow system 106 utilizes a container orchestration engine to execute the individual workflow containers arranged within the external sequencing diagnostic workflow to perform a diagnostic analysis of a sample nucleotide sequence.

As shown, in some embodiments, the diagnostic workflow system 106 performs the act 206 to execute the external sequencing diagnostic workflow after identifying sequencing data or after sequencing is complete (as performed by the variant analysis model 107). For example, the diagnostic workflow system 106 executes the external sequencing diagnostic workflow after a variant analysis performed by the variant analysis model 107 identifies or generates nucleotide base calls for a sample nucleotide sequence. For example, in some embodiments, the diagnostic workflow system 106 detects completion of sequencing analysis by the variant analysis model 107 which triggers the execution of the external sequencing diagnostic workflow automatically.

In other embodiments, the diagnostic workflow system 106 performs the act 206 to execute the external sequencing diagnostic workflow during variant analysis, or while the variant analysis model 107 is generating the nucleotide base calls. More particularly, the diagnostic workflow system 106 communicates with, or utilizes, the variant analysis model 107 while also (e.g., simultaneously or in tandem) executing the external sequencing diagnostic workflow via the container orchestration engine. For example, the diagnostic workflow system 106 generates certain nucleotide base calls required by certain workflow containers of the external sequencing diagnostic workflow, and as the variant analysis model 107 is generating other nucleotide base calls, the diagnostic workflow system 106 executes those portions of the external sequencing diagnostic workflow that are executable based on the available nucleotide base calls at that point. As sequencing progresses, the diagnostic workflow system 106 continues to execute the external sequencing diagnostic workflow until the full diagnostic analysis is complete based on a complete set of nucleotide base calls from the variant analysis model 107.

As further illustrated in FIG. 2, the diagnostic workflow system 106 performs an act 208 to provide diagnostic workflow results. In particular, the diagnostic workflow system 106 provides diagnostic workflow results generated as a result or product of the external sequencing diagnostic workflow. In some cases, the diagnostic workflow system 106 provides the diagnostic workflow results to a web hosting server to relay to an external system operated by an external entity. In other cases, the diagnostic workflow system 106 provides the diagnostic workflow results directly to an external system (e.g., via the client device 108). For example, the diagnostic workflow system 106 provides diagnostic workflow results for display via the client device 108 within a graphical user interface.

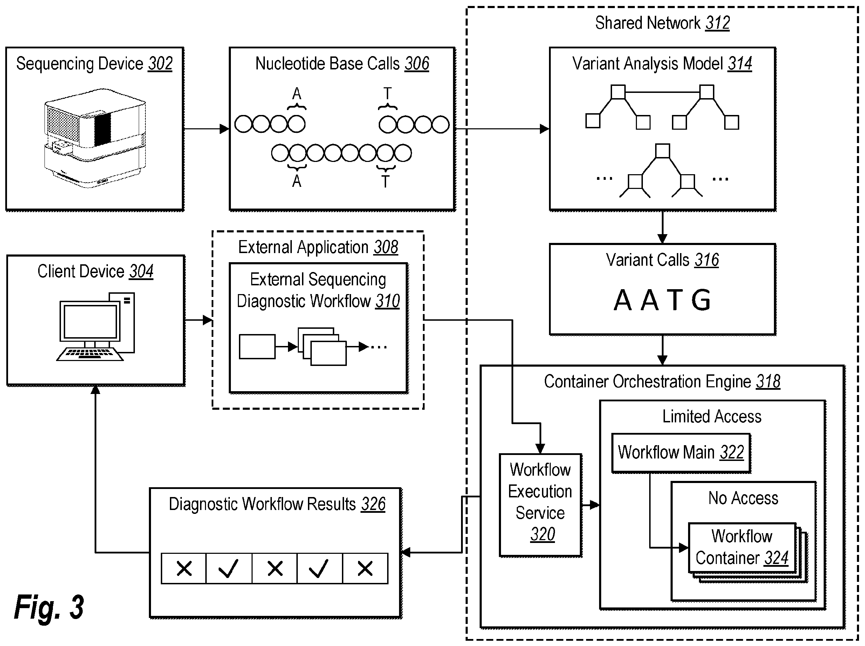

As mentioned above, in certain described embodiments, the diagnostic workflow system 106 executes an external sequencing diagnostic workflow generated by a system external to (one or more servers hosting) the diagnostic workflow system 106. In particular, the diagnostic workflow system 106 facilitates external systems to generate and execute custom sequencing diagnostic workflows for leveraging proprietary sequencing data such as nucleotide base calls generated by an internal variant analysis model (e.g., the variant analysis model 107) for their own diagnostics purposes, without risking exposure or corruption of the sequencing data. FIG. 3 illustrates an example flow for executing an external sequencing diagnostic workflow on internal sequencing data in a secure environment in accordance with one or more embodiments.

As illustrated in FIG. 3, the diagnostic workflow system 106 utilizes a container orchestration engine 318 to execute an external sequencing diagnostic workflow 310. Specifically, the diagnostic workflow system 106 executes the external sequencing diagnostic workflow 310 for performing a diagnostic analysis on nucleotide base calls 306 generated by a sequencing device 302. Indeed, as shown, the sequencing device 302 analyzes a sample nucleotide sequence to generate (e.g., via sequencing by synthesis or "SBS") the nucleotide base calls 306 for the sample sequence. In turn, the sequencing device 302 provides the nucleotide base calls 306 to (a device hosting) a variant analysis model 314, whereupon the variant analysis model 314 generates variant calls 316 from the nucleotide base calls 306.

Indeed, the variant analysis model 314 generates the variant calls 316 from the nucleotide base calls 306 and/or other sequencing data such as sequencing metrics. For example, in some cases, the sequencing device 302 generates sequencing data, such as the nucleotide base calls 306 and sequencing metrics from a sample nucleotide sequence. The diagnostic workflow system 106 can access or otherwise utilize the sequencing data (including the nucleotide base calls 306) as the basis for performing tertiary analysis or additional diagnostics (e.g., via container orchestration engine 318).

As shown, the diagnostic workflow system 106 utilizes a container orchestration engine 318 to perform additional diagnostics or tertiary analysis on the variant calls 316 (which are based on the nucleotide base calls 306 and other sequencing data at corresponding genomic coordinates). For example, the diagnostic workflow system 106 utilizes the container orchestration engine 318 to execute a sequencing diagnostic workflow such as the external sequencing diagnostic workflow 310. In one or more embodiments, the container orchestration engine 318 and the variant analysis model 314 are located on a shared network 312, such as a local area network, to retain the variant calls 316 and other sequencing data within a closed environment (e.g., for added data security). Indeed, the diagnostic workflow system 106 communicates with both the variant analysis model 314 and the container orchestration engine 318 via the shared network 312, where the variant analysis model 314 is located on one server device, and the container orchestration engine 318 is located on a different server device within the shared network 312.

In certain implementations, the diagnostic workflow system 106 implements the external sequencing diagnostic workflow 310 that is generated or modified by an external system (e.g., a third-party system). In some cases, an external system refers to a system that is external to the shared network 312 or a system that is otherwise operated by an external (e.g., third-party) entity apart from the genomic analysis platform 104. In these or other cases, the client device 304 (e.g., the client device 108) is associated with (e.g., part of) an external system. In some embodiments, the client device 304 generates or arranges the external sequencing diagnostic workflow 310 as part of an external application 308.

Along these lines, the external application 308 includes a definition of the external sequencing diagnostic workflow 310 along with workflow resource data (e.g., genomes), docker images containing software dependencies (e.g., the variant analysis model 314), and metadata such as a name and version of the external application 308 and how the external application 308 interfaces with the sequencing device 302 (e.g., compatible index adapter kits and library preparation kits) as well as how diagnostic analysis can be configured and what parameters may be specified (e.g., by the client device 304). The client device 304 either arranges workflow containers for the external sequencing diagnostic workflow 310 from the ground up or modifies a preexisting arrangement of workflow containers (e.g., as part of a workflow or application already installed on the genomic analysis platform 104) to generate the external sequencing diagnostic workflow 310 for performing a particular diagnostic.

For example, the diagnostic workflow system 106 provides, via a web interface, access to one or more internal sequencing diagnostic workflows (or applications) that comply with one or more standardized genetic diagnostic protocols or other analysis protocols. The diagnostic workflow system 106 further facilitates alterations or modifications to the sequencing diagnostic workflows (to thereby generate an external sequencing diagnostic workflow) by adding, removing, or modifying definitions of one or more workflow containers within the workflows. In addition, the diagnostic workflow system 106 facilitates generating entirely new external sequencing diagnostic workflows by arranging available workflow containers and/or by generating or defining new workflow containers.

As shown, in one or more embodiments, the diagnostic workflow system 106 receives the external sequencing diagnostic workflow 310 (e.g., as part of the external application 308) from the client device 304. In other embodiments, the diagnostic workflow system 106 receives the external sequencing diagnostic workflow 310 from another server communicating with the client device 304. The diagnostic workflow system 106 further imports the external sequencing diagnostic workflow 310 into the container orchestration engine 318 to execute the external sequencing diagnostic workflow 310 and generate diagnostic workflow results 326. Indeed, the diagnostic workflow system 106 executes the external sequencing diagnostic workflow 310 internally without additional communication to or from an external system, processing the variant calls 316 (or the nucleotide base calls 306 and/or other sequencing data) in accordance with the workflow containers defined in the external sequencing diagnostic workflow 310.

In some embodiments, the diagnostic workflow system 106 executes the external sequencing diagnostic workflow 310 to perform a diagnostic analysis on the nucleotide base calls 306 (and other sequencing data) and/or the variant calls 316. In some cases, the diagnostic workflow system 106 receives an indication (e.g., via a user interface presented on the client device 304) to perform a sequencing run. In response to the indication, the diagnostic workflow system 106 utilizes the sequencing device 302 to generate the nucleotide base calls 306 from a sample nucleotide sequence and further utilizes the variant analysis model 314 to generate the variant calls 316 from the nucleotide base calls 306. In some embodiments, the diagnostic workflow system 106 receives the nucleotide base calls 306 (and other sequencing data) from the sequencing device 302 operated at a separate server. In these or other embodiments, the diagnostic workflow system 106 receives the variant calls 316 from the variant analysis model 314 operated at a separate server. The diagnostic workflow system 106 further performs the diagnostic analysis of the external sequencing diagnostic workflow 310 either during or after the sequencing run.

As shown, the diagnostic workflow system 106 executes the external sequencing diagnostic workflow 310 via a workflow execution service 320 of the container orchestration engine 318. In particular, the diagnostic workflow system 106 utilizes a particular workflow container of the container orchestration engine 318 called a workflow execution service 320 which triggers execution of the external sequencing diagnostic workflow 310. For example, the workflow execution service 320 triggers execution by detecting that sequencing is complete (e.g., the sequencing device 302 has completed generating the nucleotide base calls 306 and/or the variant analysis model 314 has completed generating the variant calls 316 from the nucleotide base calls 306) or by receiving (e.g., via a user interface presented on the client device 304) a post defining parameters for execution of the external sequencing diagnostic workflow 310.

In some cases, the post defines parameters for implementing the external sequencing diagnostic workflow 310 such as inputs, outputs, memory allocation, and or a required version of the variant analysis model 314 compatible with the external sequencing diagnostic workflow 310. In some cases, a post includes the location of the workflow definition files (e.g., within an application directory), the location of the workflow resource files (e.g., within an application directory), the location of the sequencing input directory (e.g., a run folder), the location of an output directory, and any user configured parameters to the workflow. Indeed, based on a post, the diagnostic workflow system 106 can provide a number of functionalities for executing the external sequencing diagnostic workflow 310, including: (i) containerized task execution, (ii) orchestration of tasks, (iii) management of inputs and outputs for individual workflow containers of tasks and for overall workflows, (iv) variant analysis model (DRAGEN) acceleration, (v) automated workflow execution (e.g., sequencing to diagnostics without user interaction), (vi) system resource management (e.g., scheduling tasks for workflow containers based on CPU, RAM, storage, and FPGA resources), and (vii) workflow status.

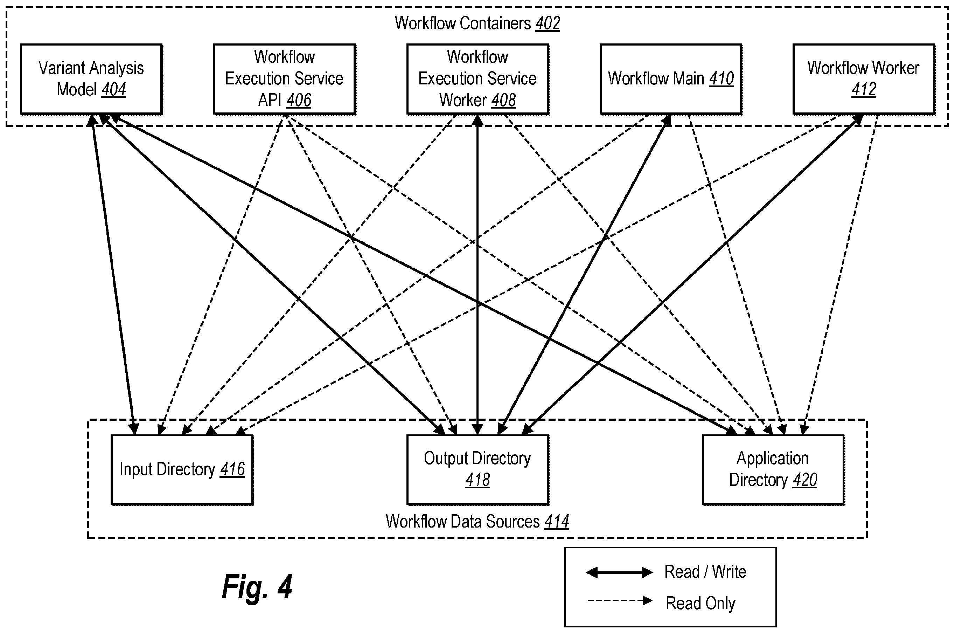

The diagnostic workflow system 106 provides further security in preventing exposure or corrupting of sequencing data such as the nucleotide base calls 306 and/or the variant calls 316. Indeed, the diagnostic workflow system 106 isolates or silos individual workflow containers of the external sequencing diagnostic workflow 310 to limit the workflow data sources they can access. As shown, the diagnostic workflow system 106 grants only limited access to sequencing data certain workflow containers and grants no access to other workflow containers. For instance, the container orchestration engine 318 utilizes a workflow main container 322 as an overall process that orchestrates the execution of the additional workflow containers such as workflow container 324 which make up the bulk of the functions for the external sequencing diagnostic workflow 310. As depicted, the diagnostic workflow system 106 grants limited access to the workflow main container 322 and no sequencing data access to the workflow container 324 (and the other task-specific workflow containers). By compartmentalizing the workflow containers and controlling data access in this way while still performing detailed diagnostics on sequencing data, the diagnostic workflow system 106 provides data security and protocol compliance for various standardized genetic diagnosis protocols such as IVD or other standardized analysis (e.g., non-diagnostic) protocols such as IUO and RUO. Additional detail regarding the data-specific isolation of workflow containers is provided below with reference to FIG. 4.

In one more embodiments, the diagnostic workflow system 106 facilitates unified runtime between cloud and edge. To elaborate, the diagnostic workflow system 106 can enable external systems to initiate runs via the variant analysis model 314 and to provide external sequencing diagnostic workflows for implementation by the container orchestration engine 318. In some cases, the diagnostic workflow system 106 can execute an external sequencing diagnostic workflow (e.g., the external sequencing diagnostic workflow 310) utilizing an edge server device or a distributed cloud server in approximately the same runtime.

In addition, the diagnostic workflow system 106 provides portability-e.g., the ability to deploy components of an external sequencing diagnostic workflow or the entire solution on different servers as well as a sequencing device (e.g., the sequencing device 302). Indeed, in some cases, the container orchestration engine 318 orchestrates execution of different workflow containers across different components such as the sequencing device 302, the variant analysis model 314, and/or various server devices. For instance, some workflow containers of the external sequencing diagnostic workflow 310 are executed by a first server device while other workflow containers are executed by another server device or by the sequencing device 302. The diagnostic workflow system 106 thus facilitates unified enablement of workflows between the cloud and edge servers. Thus, once a workflow container performs a task and writes an output, the diagnostic workflow system 106 can utilize the container orchestration engine 318 to access the output for executing another workflow container at another location within the environment (e.g., write once, run everywhere).

As mentioned above, in certain embodiments, the diagnostic workflow system 106 isolates individual workflow containers of an external sequencing diagnostic workflow to protect sequencing data. In particular, the diagnostic workflow system 106 determines which workflow containers of an external sequencing diagnostic workflow are permitted to access which workflow data sources for reading and/or writing permissions. FIG. 4 illustrates an example depiction of permissions the diagnostic workflow system 106 assigns to certain workflow containers in accordance with one or more embodiments.

As illustrated in FIG. 4, the diagnostic workflow system 106 isolates individual workflow containers within a set of workflow containers 402 of an external sequencing diagnostic workflow (e.g., the external sequencing diagnostic workflow 310). For example, the diagnostic workflow system 106 designates specific permissions for each of the workflow containers 402 to read from and/or write to certain workflow data sources 414. Thus, each granular task performed as part of an external sequencing diagnostic workflow can access only the data within a designated or permitted workflow data source.

Indeed, the diagnostic workflow system 106 can designate the workflow data sources 414 storing different types of workflow data and can activate access to one source while prevent access to another source for a given workflow container. In some cases, the diagnostic workflow system 106 mounts the workflow data sources 414 as read only for one or more workflow containers while mounting as read-write for other workflow containers. By selectively designating data access for each of the workflow containers 402, the diagnostic workflow system 106 reduces exposure of sequencing data. Consequently, the diagnostic workflow system 106 can prevent data corruption or other deleterious effects that might otherwise result from exposing sequencing data to potentially harmful sequencing diagnostic workflows.

As shown, the diagnostic workflow system 106 can implement a variant analysis model 404 (e.g., the variant analysis model 314) as a workflow container (or a group of workflow containers). Indeed, while the variant analysis model 404 may not necessarily by part of a container orchestration engine (e.g., the container orchestration engine 318), the genomic analysis platform 104 that houses the container orchestration engine 318 and the variant analysis model 404 may nevertheless utilize workflow containers for individual tasks. As indicated in FIG. 4, the diagnostic workflow system 106 permits the variant analysis model 404 to both read and write to each of the workflow data sources 414-an input directory 416, an output directory 418, and an application directory 420.

Generally, the input directory 416 stores a run folder, a sample sheet, sample mapping, and other sequencing related files including sequencing data, such as the nucleotide base calls 306, the variant calls 316 (e.g., variant call files or fields), and sequencing metrics generated by the sequencing device 302 and/or variant analysis model 404. The output directory 418 stores information such as diagnostic workflow results and intermediate outputs generated by workflow containers and input into other workflow containers. In addition, the application directory 420 stores the files specific to an application such as a workflow definition including input designations, output designations, memory allocations, compatibility metrics (e.g., indicating compatible versions of the variant analysis model 404) and definitions of various workflow containers included as part of an external sequencing diagnostic workflow.

As illustrated in FIG. 4, the diagnostic workflow system 106 isolates a workflow execution service application programming interface (API) 406. Specifically, the diagnostic workflow system 106 permits the workflow execution service API 406 read only permissions for each of the workflow data sources 414, including the input directory 416, the output directory 418, and the application directory 420. In some cases, the workflow execution service API 406 can provide access to and call various workflow containers. For example, the workflow execution service API 406 can initiate execution of an external sequencing diagnostic workflow based on a post provided by a client device (e.g., the client device 304).

In addition, the diagnostic workflow system 106 isolates a workflow execution service worker 408 (e.g., the workflow execution service 320). Specifically, the diagnostic workflow system 106 permits the workflow execution service worker 408 to read only from both the input directory 416 and the application directory 420. The diagnostic workflow system 106 further permits the workflow execution service worker 408 to read and write to the output directory 418. Indeed, the workflow execution service worker 408 orchestrates implementation of additional workflow containers for an external sequencing diagnostic workflow and further writes the diagnostic workflow results to the output directory 418.

Further, the diagnostic workflow system 106 isolates a workflow main container 410. Specifically, like the workflow execution service worker 408, the diagnostic workflow system 106 permits the workflow main container 410 to read only from the input directory 416 and the application directory 420. The diagnostic workflow system 106 further permits the workflow main container 410 to read and write to the output directory 418.

As further illustrated in FIG. 4, the diagnostic workflow system 106 isolates a workflow worker container 412 (e.g., the workflow container 324). Specifically, like the workflow execution service worker 408 and the workflow main container 410, the diagnostic workflow system 106 permits the workflow worker container 412 to read only from the input directory 416 and the application directory 420. The diagnostic workflow system 106 further permits the workflow worker container 412 to read and write to the output directory 418.

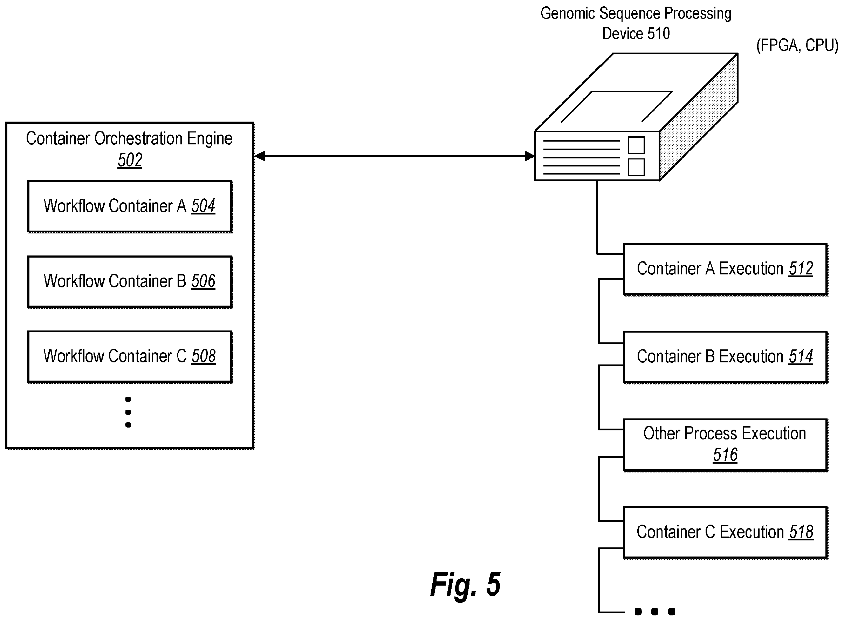

As mentioned above, in certain embodiments, the diagnostic workflow system 106 utilizes a container orchestration engine to orchestrate execution of workflow containers (e.g., for individual tasks). In particular, the diagnostic workflow system 106 utilizes a container orchestration engine to schedule available computing resources for performing tasks of workflow containers. FIG. 5 illustrates utilizing a container orchestration engine to schedule execution of workflow containers in accordance with one or more embodiments.

As illustrated in FIG. 5, the diagnostic workflow system 106 utilizes a container orchestration engine 502 (e.g., the container orchestration engine 318) to execute workflow containers of a sequencing diagnostic workflow (e.g., the external sequencing diagnostic workflow 310). In particular, the container orchestration engine 502 orchestrates performance of workflow container A 504, workflow container B 506, and workflow container C 508 by way of a genomic sequence processing device (e.g., an FPGA, or a CPU). The depiction of FIG. 5 is an example, and in some cases, the container orchestration engine 502 orchestrates performance of different workflow containers across different servers or devices with different available resources, where some may utilize CPUs and others may utilize FPGAs (or other computing processors).

In scheduling the performance of the workflow containers, the container orchestration engine 502 determines available computing resources of the genomic sequence processing device 510 (e.g., an FPGA, such as a DRAGEN FPGA or a CPU). Available resources can fluctuate over time as the genomic sequence processing device 510 performs different tasks either from the container orchestration engine 502 or from another source. Thus, the diagnostic workflow system 106 utilizes the container orchestration engine 502 to perform adaptive, elastic resource allocation and to scale with available resources such as CPU, RAM, FPGA resources, variant analysis model (DRAGEN) accelerators, and a number of available worker nodes.

As shown, the container orchestration engine 502 communicates with the genomic sequence processing device 510 to perform container A execution 512 to execute the workflow container A 504. Indeed, based on determining that the genomic sequence processing device 510 has available computing resources such as an available worker node, available RAM, available processing power, and/or available FPGA processing capacity, the container orchestration engine 502 provides workflow container A 504. In turn, the genomic sequence processing device 510 performs the container A execution 512. In some embodiments, container A execution 512 produces an intermediate container output which the diagnostic workflow system 106 stores in a network location (e.g., the input directory 416, the output directory 418, or the application directory 420) for use as an input for a subsequent workflow container.

As further illustrated in FIG. 5, the container orchestration engine 502 provides workflow container B 506 and workflow container C 508 for execution by the genomic sequence processing device 510. Indeed, the container orchestration engine 502 determines or detects when container A execution 512 completes and then provides workflow container B 506 when the genomic sequence processing device 510 is available. In turn, the genomic sequence processing device 510 performs the container B execution 514.

In some cases, the genomic sequence processing device 510 is an FPGA that executes processes in series (e.g., that cannot execute multiple workflow containers simultaneously). For example, the genomic sequence processing device 510 implements a variant analysis model (e.g., the variant analysis model 404) to generate nucleotide base calls and other sequencing data by performing operations in sequence. Thus, in some cases, the genomic sequence processing device 510 is busy performing an operation and cannot perform another until the operation completes. The container orchestration engine 502, therefore, schedules resources of the genomic sequence processing device 510 to perform tasks of workflow containers based on availability.

In some embodiments, the genomic sequence processing device 510 performs other tasks not related to a sequencing diagnostic workflow (or for another sequencing diagnostic workflow). Thus, the container orchestration engine 502 orchestrates execution of the workflow containers 504-508 based on availability of the genomic sequence processing device 510. As shown, the genomic sequence processing device 510 performs the other process execution 516 (not related to a sequencing diagnostic workflow) after the container B execution 514 and before the container C execution. Indeed, the container orchestration engine 502 determines that the genomic sequence processing device 510 is available once the other process execution 516 completes and provides workflow container C 508 to the genomic sequence processing device 510 for execution. In turn, the genomic sequence processing device 510 performs the container C execution 518.

The container orchestration engine 502 continues to orchestrate performance of workflow containers for a sequencing diagnostic workflow (e.g., an external sequencing diagnostic workflow) until it is complete. For example, the container orchestration engine 502 utilizes the genomic sequence processing device 510 and/or other processor devices to execute various workflow containers until all workflow containers are executed (in their proper order as defined by the workflow). The container orchestration engine 502 further generates a diagnostic workflow result from the overall workflow. The diagnostic workflow system 106 can provide the result for display within a user interface on a client device (e.g., the client device 304). By scheduling resource allocation to execute workflow containers, the diagnostic workflow system 106 (via the container orchestration engine 502) can efficiently utilize computing resources by reducing downtime and preventing overlapping processes (especially for FPGAs or other series-oriented devices) to reduce crashes and slowdowns.

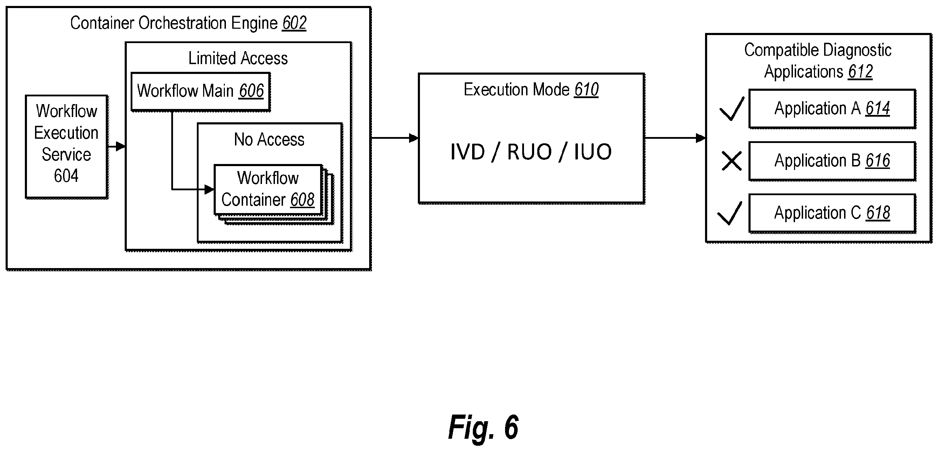

As mentioned above, in certain embodiments, the diagnostic workflow system 106 determines an execution mode of a container orchestration engine. In particular, the diagnostic workflow system 106 determines an execution mode based on a standardized genetic diagnostic protocol that the container orchestration engine aims to satisfy when executing a particular workflow (e.g., an external sequencing diagnostic workflow). FIG. 6 illustrates an example flow for determining or identifying diagnostic applications that are compatible (and/or diagnostic applications that are incompatible) with an execution mode of a container orchestration engine in accordance with one or more embodiments.

As illustrated in FIG. 6, the diagnostic workflow system 106 utilizes a container orchestration engine 602 to execute diagnostic workflow that includes various workflow containers such as the workflow container 608. Indeed, as described, the container orchestration engine 602 utilizes the workflow execution service 604 and a workflow main container 606 to trigger and facilitate execution of the workflow. In some embodiments, an external sequencing diagnostic workflow (or its parent application) designates a particular execution mode 610 for the container orchestration engine 602. For example, the workflow indicates a standardized genetic diagnostic protocol such as IVD or indicates another standardized analysis protocol, such as IUO or RUO that the container orchestration engine 602 must comply with when executing a workflow.

Based on detecting or determining an execution mode 610 for the container orchestration engine 602, the diagnostic workflow system 106 identifies a set of compatible diagnostic applications 612. For example, the diagnostic workflow system 106 identifies diagnostic applications or workflows that have been approved by a particular regulatory body (e.g., the FDA) to satisfy a standardized genetic diagnostic protocol such as IVD. The diagnostic workflow system 106 further removes, or prevents access to, other diagnostic applications that do not satisfy the standardized genetic diagnostic protocol (e.g., applications that satisfy a lower bar such as IUO or RUO but not IVD). As another example, the diagnostic workflow system 106 identifies analysis applications that (at least) satisfy a lower bar such as IUO or RUO (including applications that satisfy higher bars, while preventing access to other applications or workflows that do not). Thus, the diagnostic workflow system 106 ensures that only the compatible diagnostic applications 612 that are compatible with the execution mode 610 are available for a particular sequencing run. As shown, the diagnostic workflow system 106 determines that Application A 614 and Application C 618 are compatible with the execution mode 610 of the container orchestration engine 602, while Application B 616 is incompatible with the execution mode 610.

The diagnostic workflow system 106 further grants or permits access to the compatible diagnostic applications 612. For example, the diagnostic workflow system 106 grants access to a client device (e.g., the client device 304) for selecting and applying (e.g., by presenting via a user interface) one or more applications or workflows to a sequencing run executed in the execution mode 610. In some cases, the diagnostic workflow system 106 grants access for the container orchestration engine 602 to execute all or part of (e.g., particular workflow containers within) one or more of the compatible diagnostic applications 612. For instance, as part of executing an external sequencing diagnostic workflow, the diagnostic workflow system 106 can access only those applications that comply with the standardized genetic diagnostic protocol. In some cases, the diagnostic workflow system 106 grants access to individual workflow containers within the compatible diagnostic applications 612 for application or adaptation into an external sequencing diagnostic workflow implemented by the container orchestration engine 602.

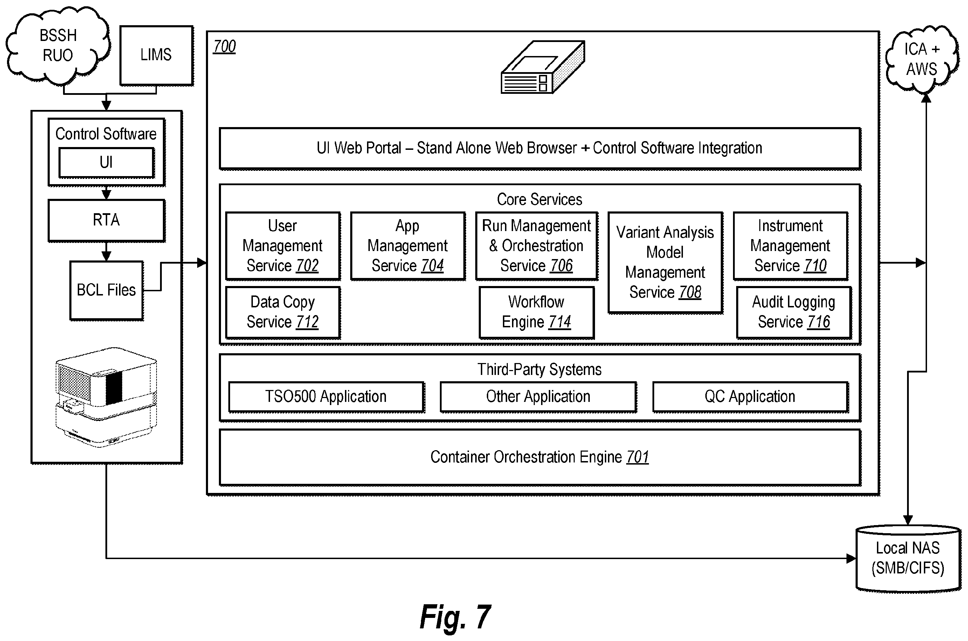

As mentioned above, in certain embodiments, the diagnostic workflow system 106 utilizes containers and pods to execute external workflows associated with nucleotide reads and base calls of a sample sequence. In particular, the diagnostic workflow system 106 can analyze sequencing data via a diagnostic workflow to identify genetic markers or hereditary traits indicated within a genomic sample. FIG. 7 illustrates an example diagram of components, applications, devices, and containers of a system architecture (e.g., installed on a local server device) involved in implementing an external diagnostic workflow in accordance with one or more embodiments.

As illustrated in FIG. 7, the diagnostic workflow system 106 utilizes a sequencing device (e.g., the sequencing device 114) to communicate with various components or systems and to perform sequencing operations used by, and/or as instructed by, a version of a variant analysis model on a local server (e.g., the variant analysis model 107 on the server device(s) 102). For example, the diagnostic workflow system 106 communicates with a BaseSpace Sequencing Hub ("BSSH") or cloud-based interface for research use only ("RUO") and a lab information management system ("LIMS") to generate base calls and other sequencing data for nucleotide bases of a genomic sample.

Based on information from the BSSH RUO and/or the LIMS, the diagnostic workflow system 106 performs a real-time analysis ("RTA") of a sample. More specifically, the diagnostic workflow system 106 performs RTA to determine base calls, variant calls, and/or various metrics from nucleotide bases of a genomic sample according to a sequencing plan. Based on the RTA, the diagnostic workflow system 106 generates a binary base call ("BCL") file that includes raw data generated and output by one or more sequencing runs (e.g., via the RTA). Indeed, the BCL file can indicate base calls, variant calls, and/or other sequencing information for interpretation by a variant analysis model and/or some other system.

To organize or plan a sequencing run of the RTA, the diagnostic workflow system 106 provides control software (e.g., including a user interface) for planning or scheduling a sequencing run on a particular sample. Indeed, the diagnostic workflow system 106 provides control software and a user interface for planning one or more sequencing runs to, for example, test a genomic sample for a particular genetic marker according to plan parameters. For instance, the control software enables a user to specify parameters for a sequencing run and/or to test for specific markers. As shown, the diagnostic workflow system 106 can integrate the control software for the sequencing device with a user interface web portal (which includes a standalone web browser and control software integration) to interface with the sequencing device for planning a sequencing run.

In some cases, the diagnostic workflow system 106 facilitates local planning for a sequencing run, where the planning software (e.g., the control software) is hosted by a local server device, such as a local edge server. In these or other cases, the diagnostic workflow system 106 facilitates cloud planning for a sequencing run, where the planning software (e.g., the control software) is hosted on a cloud server rather than a local server. In a similar fashion, the execution of a variant analysis model can be local or cloud-based as well, depending on whether the server hosting the variant analysis model is a local server (e.g., the server device(s) 102). Accordingly, (i) the diagnostic workflow system 106 can be executed either locally on a local server device located at or near the sequencing device 114 or remotely on a cloud-based server device in combination with (ii) the variant analysis model 107 executed either locally on a local server device located at or near the sequencing device 114 or remotely on a cloud-based server device.

As further illustrated in FIG. 7, the system architecture 700 of the diagnostic workflow system 106 includes, or communicates with, containers or systems associated with one or more core services. Indeed, as shown, the diagnostic workflow system 106 includes the services of the system architecture 700. To manage or orchestrate the various services of the system architecture 700, the system architecture 700 includes a container orchestration engine 701 (e.g., K3S or Kubernetes) to manage and implement various pods and containers associated with performing genomic analyses via diagnostic workflows as described herein. As described, the diagnostic workflow system 106 utilizes a container orchestration engine 701 to orchestrate or coordinate a diagnostic workflow to analyze a genomic sequence for base calling, variant calling (e.g., as indicated by an application of a third-party system). The container orchestration engine 701 includes pods and containers to perform other functions as well, including user management, application management, run management, variant analysis model management, instrument management, data copying, and audit logging.

For example, the system architecture 700 includes a user management service 702 that includes a set of one or more user management pods or containers. The user management service 702 performs various processes or functions for providing a single sign-on ("SSO") experience system wide. Specifically, the user management service 702 can include one or more containers or pods that include or access user information for a third-party system to, for example, determine a diagnostic workflow (e.g., from one of the third-party systems) for analyzing a genomic sequence, including user settings or preferences for executing the diagnostic workflow. Based on the determination of the diagnostic workflow and/or the user settings, the user management service 702 can communicate with other services of the system architecture 700 to initiate performance of the diagnostic workflow to analyze a genomic sequence accordingly.

In addition, the system architecture 700 includes or utilizes an application management service 704 in communication with the container orchestration engine 701. For example, the application management service 704 manages application package installation for diagnostic workflows. In some cases, the application management service 704 further includes a resource manager. The resource manager can access or utilize a genomic analysis device resource as specified by an application specification and/or as part of a diagnostic workflow. To elaborate, the resource manager identifies a resource label to access a designated resource, such as an FPGA or a CPU, as a schedulable resource for access via the container orchestration engine. Indeed, in some cases, the application management service 704 includes (or receives from a third-party system) an application specification that indicates an FPGA or a CPU or some other genomic analysis device for executing a diagnostic workflow of a genomic analysis application (or a particular workflow pod), and the resource manager therefore accesses or communicates with the specified device (or other resource) for facilitating execution of the genomic analysis application (or the particular workflow pod).

As further shown, the system architecture 700 includes or utilizes a run management and orchestration service 706. To elaborate, the run management and orchestration service 706 includes one or more containers or pods for facilitating and executing genomic analysis via a diagnostic workflow, such as a sequencing run, a primary analysis, a secondary analysis, or a tertiary analysis. Indeed, the run management and orchestration service 706 includes computer code or instructions for executing a sequencing run (and/or further analysis) according to an installed version of a variant analysis model. For instance, the run management and orchestration service 706 communicates with the workflow engine 714 to execute a custom diagnostic workflow for an application, such as an application associated with a third-party system (e.g., an oncology assay application, such as TSO500 application; a QC application; or another application). The run management and orchestration service 706 further includes code for communicating with the data copy service 712 to copy input and output sequencing data (e.g., from a BCL file generated by a sequencing device) for performing a genomic analysis and/or for storing in a database, such as a local network attached storage ("NAS"), server message block ("SMB"), or common internet file system ("CIFS").

In addition, the system architecture 700 includes a variant analysis model management service 708. In particular, the variant analysis model management service 708 includes one or more containers or pods for managing a variant analysis model (e.g., the variant analysis model 107) for performing genomic analysis. For example, the variant analysis model management service 708 implements a particular diagnostic workflow using a variant analysis model to detect a genetic marker for a certain condition within a sample genomic sequence. In addition, the variant analysis model management service 708 manages model peripherals, such as licensing, self-testing, and version authentication for a variant analysis model.

As further illustrated in FIG. 7, the system architecture 700 includes an instrument management service 710. In one or more embodiments, the instrument management service 710 includes one or more containers or pods for pairing and monitoring instruments used as part of a sequencing workflow and/or a genomic analysis workflow after sequencing. For instance, the instrument management service 710 manages instruments of a sequencing device and/or a variant analysis model to pair compatible instruments with indicated versions of a variant analysis model (or vice-versa). The system architecture 700 further includes an audit logging service 716 for monitoring and logging performance of instruments, components of a variant analysis model, and/or containers within an application workflow. For instance, the audit logging service 716 detects and logs errors or other auditing information associated with the system architecture 700.

As suggested above in the description of FIG. 1 and elsewhere, using the system architecture 700 illustrated in FIG. 7, the diagnostic workflow system 106 can be deployed locally on an edge server (e.g., the local server device(s) 102) or in the cloud such as on cloud-based servers hosting Illumina Connected Analytics ("ICA") and/or cloud-based servers from Amazon Web Services ("AWS"). For example, the diagnostic workflow system 106 can be executed locally on a local server device-or remotely on a cloud-based server device-as part of planning software that plans resources based on user input for sequencing runs or other assays, and the variant analysis model 107 can likewise be executed locally on the local server device-or remotely on a cloud-based server device-to analyze BCL data and determine variant calls or other metrics.

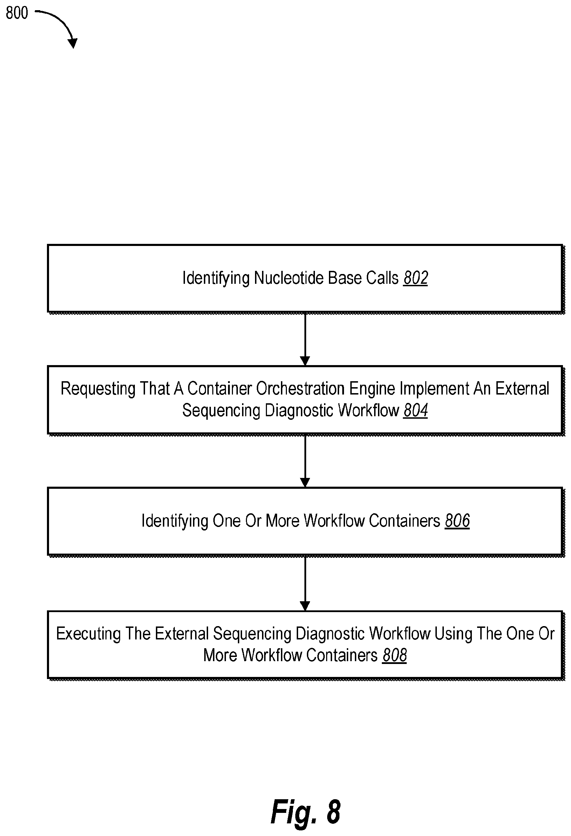

Turning now to FIG. 8, this figure illustrates an example flowchart of a series of acts of executing an external sequencing diagnostic workflow utilizing a container orchestration engine in accordance with one or more embodiments. While FIG. 8 illustrates acts according to one embodiment, alternative embodiments may omit, add to, reorder, and/or modify any of the acts shown in FIG. 8. The acts of FIG. 8 can be performed as part of a method. Alternatively, a non-transitory computer readable storage medium can comprise instructions that, when executed by one or more processors, cause a computing device to perform the acts depicted in FIG. 8. In still further embodiments, a system comprising at least one processor and a non-transitory computer readable medium comprising instructions that, when executed by one or more processors, cause the system to perform the acts of FIG. 8.

As shown in FIG. 8, the series of acts 800 includes an act 802 of identifying nucleotide base calls. In particular, the act 802 can include identifying nucleotide base calls generated by a variant analysis model for a sample nucleotide sequence.

In addition, the series of acts 800 includes an act 804 of requesting that a container orchestration engine implement an external sequencing diagnostic workflow. In particular, the act 804 can include requesting that a container orchestration engine associated with the variant analysis model implement an external sequencing diagnostic workflow for performing a diagnostic analysis on the nucleotide base calls for the sample nucleotide sequence. For example, the act 804 can involve implementing the external sequencing diagnostic workflow identified from an external application that is separate from the container orchestration engine and the variant analysis model.

As further illustrated in FIG. 8, the series of acts 800 includes an act 806 of identifying one or more workflow containers. In particular, the act 806 can include identifying one or more workflow containers associated with respective functionalities of the external sequencing diagnostic workflow.

Further, the series of acts 800 includes an act 808 of executing the external sequencing diagnostic workflow using the one or more workflow containers. In particular, the act 808 can include executing the external sequencing diagnostic workflow by utilizing the container orchestration engine to implement the one or more workflow containers. For example, the act 808 can involve executing the external sequencing diagnostic workflow after a variant analysis by the variant analysis model generates the nucleotide base calls for the sample nucleotide sequence or during the variant analysis by the variant analysis model. In some cases, the act 808 involves selectively preventing the one or more workflow containers from accessing sequencing data of the sample nucleotide sequence while executing the external sequencing diagnostic workflow. In these or other cases, the act 808 involves utilizing the container orchestration engine located on a server device hosting the variant analysis model. Indeed, the act 808 can involve executing the external sequencing diagnostic workflow generated by an external system separate from the container orchestration engine and the variant analysis model.

In some embodiments, the series of acts 800 includes an act of determining a diagnostic execution mode corresponding to a standardized genetic diagnostic protocol. The series of acts 800 can also include an act of granting, for a client device, access only to diagnostic applications compatible with the diagnostic execution mode. In certain cases, the series of acts 800 includes an act of receiving the external sequencing diagnostic workflow generated by an external device operated by an external entity.

The series of acts 800 can include an act of controlling access to different workflow data sources for the one or more workflow containers to prevent access to sequencing data. For instance, the series of acts 800 can include an act of preventing a workflow container of the one or more workflow containers from accessing sequencing data of the sample nucleotide sequence. Preventing a workflow container from accessing sequencing data of the sample nucleotide sequence can involve preventing the workflow container from accessing one or more workflow data sources comprising an input directory, an output directory, and an application directory.

The series of acts 800 can also include an act of receiving an indication of a label defining a version of the variant analysis model and a memory allocation to utilize for executing the external sequencing diagnostic workflow. Additionally, the series of acts 800 can include an act of designating a plurality of workflow data sources storing different types of workflow data. Further, the series of acts 800 can include an act of activating, for a workflow container from among the one or more workflow containers, access to a first workflow data source of the plurality of workflow data sources while preventing access to other workflow data sources of the plurality of workflow data sources. The series of acts 800 can include an act of mounting a plurality of Workflow data sources as read only for the one or more workflow containers. In some embodiments, the series of acts 800 includes an act of triggering execution of the external sequencing diagnostic workflow by receiving, via the container orchestration engine, a post defining parameters for implementing the external sequencing diagnostic workflow.

In some embodiments, the series of acts 800 includes an act of satisfying one or more standardized genetic diagnostic protocols while also executing the external sequencing diagnostic workflow by encoding a workflow execution application to grant the external sequencing diagnostic workflow read-only access to sequencing data associated with the nucleotide base calls of the sample nucleotide sequence during execution of the external sequencing diagnostic workflow.

The methods described herein can be used in conjunction with a variety of nucleic acid sequencing techniques. Particularly applicable techniques are those wherein nucleic acids are attached at fixed locations in an array such that their relative positions do not change and wherein the array is repeatedly imaged. Embodiments in which images are obtained in different color channels, for example, coinciding with different labels used to distinguish one nucleotide base type from another are particularly applicable. In some embodiments, the process to determine the nucleotide sequence of a target nucleic acid (i.e., a nucleic acid polymer) can be an automated process. Preferred embodiments include sequencing-by-synthesis (SBS) techniques.

SBS techniques generally involve the enzymatic extension of a nascent nucleic acid strand through the iterative addition of nucleotides against a template strand. In traditional methods of SBS, a single nucleotide monomer may be provided to a target nucleotide in the presence of a polymerase in each delivery. However, in the methods described herein, more than one type of nucleotide monomer can be provided to a target nucleic acid in the presence of a polymerase in a delivery.

SBS can utilize nucleotide monomers that have a terminator moiety or those that lack any terminator moieties. Methods utilizing nucleotide monomers lacking terminators include, for example, pyrosequencing and sequencing using γ-phosphate-labeled nucleotides, as set forth in further detail below. In methods using nucleotide monomers lacking terminators, the number of nucleotides added in each cycle is generally variable and dependent upon the template sequence and the mode of nucleotide delivery. For SBS techniques that utilize nucleotide monomers having a terminator moiety, the terminator can be effectively irreversible under the sequencing conditions used as is the case for traditional Sanger sequencing which utilizes dideoxynucleotides, or the terminator can be reversible as is the case for sequencing methods developed by Solexa (now Illumina, Inc.).

SBS techniques can utilize nucleotide monomers that have a label moiety or those that lack a label moiety. Accordingly, incorporation events can be detected based on a characteristic of the label, such as fluorescence of the label; a characteristic of the nucleotide monomer such as molecular weight or charge; a byproduct of incorporation of the nucleotide, such as release of pyrophosphate; or the like. In embodiments, where two or more different nucleotides are present in a sequencing reagent, the different nucleotides can be distinguishable from each other, or alternatively, the two or more different labels can be the indistinguishable under the detection techniques being used. For example, the different nucleotides present in a sequencing reagent can have different labels and they can be distinguished using appropriate optics as exemplified by the sequencing methods developed by Solexa (now Illumina, Inc.).

Preferred embodiments include pyrosequencing techniques. Pyrosequencing detects the release of inorganic pyrophosphate (Ppi) as particular nucleotides are incorporated into the nascent strand (Ronaghi, M., Karamohamed, S., Pettersson, B., Uhlen, M. and Nyren, P. (1996) "Real-time DNA sequencing using detection of pyrophosphate release." Analytical Biochemistry 242(1), 84-9; Ronaghi, M. (2001) "Pyrosequencing sheds light on DNA sequencing." Genome Res. 11(1), 3-11; Ronaghi, M., Uhlen, M. and Nyren, P. (1998) "A sequencing method based on real-time pyrophosphate." Science 281(5375), 363; U.S. Pat. No. 6,210,891; U.S. Pat. No. 6,258,568 and U.S. Pat. No. 6,724,320. In pyrosequencing, released Ppi can be detected by being immediately converted to adenosine triphosphate (ATP) by ATP sulfurylase, and the level of ATP generated is detected via luciferase-produced photons. The nucleic acids to be sequenced can be attached to features in an array and the array can be imaged to capture the chemiluminescent signals that are produced due to incorporation of a nucleotides at the features of the array. An image can be obtained after the array is treated with a particular nucleotide type (e.g., A, T, C or G). Images obtained after addition of each nucleotide type will differ with regard to which features in the array are detected. These differences in the image reflect the different sequence content of the features on the array. However, the relative locations of each feature will remain unchanged in the images. The images can be stored, processed and analyzed using the methods set forth herein. For example, images obtained after treatment of the array with each different nucleotide type can be handled in the same way as exemplified herein for images obtained from different detection channels for reversible terminator-based sequencing methods.

In another exemplary type of SBS, cycle sequencing is accomplished by stepwise addition of reversible terminator nucleotides containing, for example, a cleavable or photobleachable dye label as described, for example, in WO 04/018497 and U.S. Pat. No. 7,057,026. This approach is being commercialized by Solexa (now Illumina Inc.), and is also described in WO 91/06678 and WO 07/123,744. The availability of fluorescently-labeled terminators in which both the termination can be reversed and the fluorescent label cleaved facilitates efficient cyclic reversible termination (CRT) sequencing. Polymerases can also be co-engineered to efficiently incorporate and extend from these modified nucleotides.

Preferably in reversible terminator-based sequencing embodiments, the labels do not substantially inhibit extension under SBS reaction conditions. However, the detection labels can be removable, for example, by cleavage or degradation. Images can be captured following incorporation of labels into arrayed nucleic acid features. In particular embodiments, each cycle involves simultaneous delivery of four different nucleotide types to the array and each nucleotide type has a spectrally distinct label. Four images can then be obtained, each using a detection channel that is selective for one of the four different labels. Alternatively, different nucleotide types can be added sequentially and an image of the array can be obtained between each addition step. In such embodiments, each image will show nucleic acid features that have incorporated nucleotides of a particular type. Different features are present or absent in the different images due the different sequence content of each feature. However, the relative position of the features will remain uncharged in the images. Images obtained from such reversible terminator-SBS methods can be stored, processed and analyzed as set forth herein. Following the image capture step, labels can be removed and reversible terminator moieties can be removed for subsequent cycles of nucleotide addition and detection. Removal of the labels after they have been detected in a particular cycle and prior to a subsequent cycle can provide the advantage of reducing background signal and crosstalk between cycles. Examples of useful labels and removal methods are set forth below.

In particular embodiments some or all of the nucleotide monomers can include reversible terminators. In such embodiments, reversible terminators/cleavable fluors can include fluor linked to the ribose moiety via a 3' ester linkage (Metzker, Genome Res. 15:1767-1776 (2005). Other approaches have separated the terminator chemistry from the cleavage of the fluorescence label (Ruparel et al., Proc Natl Acad Sci USA 102: 5932-7 (2005). Ruparel et al described the development of reversible terminators that used a small 3' allyl group to block extension, but could easily be deblocked by a short treatment with a palladium catalyst. The fluorophore was attached to the base via a photocleavable linker that could easily be cleaved by a 30 second exposure to long wavelength UV light. Thus, either disulfide reduction or photocleavage can be used as a cleavable linker. Another approach to reversible termination is the use of natural termination that ensues after placement of a bulky dye on a dNTP. The presence of a charged bulky dye on the dNTP can act as an effective terminator through steric and/or electrostatic hindrance. The presence of one incorporation event prevents further incorporations unless the dye is removed. Cleavage of the dye removes the fluor and effectively reverses the termination. Examples of modified nucleotides are also described in U.S. Pat. No. 7,427,673, and U.S. Pat. No. 7,057,026.

Additional exemplary SBS systems and methods which can be utilized with the methods and systems described herein are described in U.S. Patent Application Publication No. 2007/0166705, U.S. Patent Application Publication No. 2006/0188901, U.S. Pat. No. 7,057,026, U.S. Patent Application Publication No. 2006/0240439, U.S. Patent Application Publication No. 2006/0281109, PCT Publication No. WO 05/065814, U.S. Patent Application Publication No. 2005/0100900, PCT Publication No. WO 06/064199, PCT Publication No. WO 07/010,251, U.S. Patent Application Publication No. 2012/0270305 and U.S. Patent Application Publication No. 2013/0260372.

Some embodiments can utilize detection of four different nucleotides using fewer than four different labels. For example, SBS can be performed utilizing methods and systems described in of U.S. Patent Application Publication No. 2013/0079232. As a first example, a pair of nucleotide types can be detected at the same wavelength, but distinguished based on a difference in intensity for one member of the pair compared to the other, or based on a change to one member of the pair (e.g. via chemical modification, photochemical modification or physical modification) that causes apparent signal to appear or disappear compared to the signal detected for the other member of the pair. As a second example, three of four different nucleotide types can be detected under particular conditions while a fourth nucleotide type lacks a label that is detectable under those conditions, or is minimally detected under those conditions (e.g., minimal detection due to background fluorescence, etc.). Incorporation of the first three nucleotide types into a nucleic acid can be determined based on presence of their respective signals and incorporation of the fourth nucleotide type into the nucleic acid can be determined based on absence or minimal detection of any signal. As a third example, one nucleotide type can include label(s) that are detected in two different channels, whereas other nucleotide types are detected in no more than one of the channels. The aforementioned three exemplary configurations are not considered mutually exclusive and can be used in various combinations. An exemplary embodiment that combines all three examples, is a fluorescent-based SBS method that uses a first nucleotide type that is detected in a first channel (e.g. dATP having a label that is detected in the first channel when excited by a first excitation wavelength), a second nucleotide type that is detected in a second channel (e.g. dCTP having a label that is detected in the second channel when excited by a second excitation wavelength), a third nucleotide type that is detected in both the first and the second channel (e.g. dTTP having at least one label that is detected in both channels when excited by the first and/or second excitation wavelength) and a fourth nucleotide type that lacks a label that is not, or minimally, detected in either channel (e.g. dGTP having no label).

Further, as described in U.S. Patent Application Publication No. 2013/0079232, sequencing data can be obtained using a single channel. In such so-called one-dye sequencing approaches, the first nucleotide type is labeled but the label is removed after the first image is generated, and the second nucleotide type is labeled only after a first image is generated. The third nucleotide type retains its label in both the first and second images, and the fourth nucleotide type remains unlabeled in both images.

Some embodiments can utilize sequencing by ligation techniques. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. As with other SBS methods, images can be obtained following treatment of an array of nucleic acid features with the labeled sequencing reagents. Each image will show nucleic acid features that have incorporated labels of a particular type. Different features are present or absent in the different images due the different sequence content of each feature, but the relative position of the features will remain unchanged in the images. Images obtained from ligation-based sequencing methods can be stored, processed and analyzed as set forth herein. Exemplary SBS systems and methods which can be utilized with the methods and systems described herein are described in U.S. Pat. No. 6,969,488, U.S. Pat. No. 6,172,218, and U.S. Pat. No. 6,306,597.

Some embodiments can utilize nanopore sequencing (Deamer, D. W. & Akeson, M. "Nanopores and nucleic acids: prospects for ultrarapid sequencing." Trends Biotechnol. 18, 147-151 (2000); Deamer, D. and D. Branton, "Characterization of nucleic acids by nanopore analysis". Acc. Chem. Res. 35:817-825 (2002); Li, J., M. Gershow, D. Stein, E. Brandin, and J. A. Golovchenko, "DNA molecules and configurations in a solid-state nanopore microscope" Nat. Mater. 2:611-615 (2003). In such embodiments, the target nucleic acid passes through a nanopore. The nanopore can be a synthetic pore or biological membrane protein, such as α-hemolysin. As the target nucleic acid passes through the nanopore, each base-pair can be identified by measuring fluctuations in the electrical conductance of the pore. (U.S. Pat. No. 7,001,792; Soni, G. V. & Meller, "A. Progress toward ultrafast DNA sequencing using solid-state nanopores." Clin. Chem. 53, 1996-2001 (2007); Healy, K. "Nanopore-based single-molecule DNA analysis." Nanomed. 2, 459-481 (2007); Cockroft, S. L., Chu, J., Amorin, M. & Ghadiri, M. R. "A single-molecule nanopore device detects DNA polymerase activity with single-nucleotide resolution." J. Am. Chem. Soc. 130, 818-820 (2008). Data obtained from nanopore sequencing can be stored, processed and analyzed as set forth herein. In particular, the data can be treated as an image in accordance with the exemplary treatment of optical images and other images that is set forth herein.

Some embodiments can utilize methods involving the real-time monitoring of DNA polymerase activity. Nucleotide incorporations can be detected through fluorescence resonance energy transfer (FRET) interactions between a fluorophore-bearing polymerase and γ-phosphate-labeled nucleotides as described, for example, in U.S. Pat. No. 7,329,492 and U.S. Pat. No. 7,211,414 or nucleotide incorporations can be detected with zero-mode waveguides as described, for example, in U.S. Pat. No. 7,315,0 and using fluorescent nucleotide analogs and engineered polymerases as described, for example, in U.S. Pat. No. 7,405,281 and U.S. Patent Application Publication No. 2008/0109082**.** The illumination can be restricted to a zeptoliter-scale volume around a surface-tethered polymerase such that incorporation of fluorescently labeled nucleotides can be observed with low background (Levene, M. J. et al. "Zero-mode waveguides for single-molecule analysis at high concentrations." Science 299, 682-686 (2003); Lundquist, P. M. et al. "Parallel confocal detection of single molecules in real time." Opt. Lett. 33, 1026-1028 (2008); Korlach, J. et al. "Selective aluminum passivation for targeted immobilization of single DNA polymerase molecules in zero-mode waveguide nano structures." Proc. Natl. Acad. Sci. USA 105, 1176-1181 (2008). Images obtained from such methods can be stored, processed and analyzed as set forth herein.

Some SBS embodiments include detection of a proton released upon incorporation of a nucleotide into an extension product. For example, sequencing based on detection of released protons can use an electrical detector and associated techniques that are commercially available from Ion Torrent (Guilford, CT, a Life Technologies subsidiary) or sequencing methods and systems described in US 2009/0026082 A1; US 2009/0127589 A1; US 2010/0137143 A1; or US 2010/0282617 A1. Methods set forth herein for amplifying target nucleic acids using kinetic exclusion can be readily applied to substrates used for detecting protons. More specifically, methods set forth herein can be used to produce clonal populations of amplicons that are used to detect protons.

The above SBS methods can be advantageously carried out in multiplex formats such that multiple different target nucleic acids are manipulated simultaneously. In particular embodiments, different target nucleic acids can be treated in a common reaction vessel or on a surface of a particular substrate. This allows convenient delivery of sequencing reagents, removal of unreacted reagents and detection of incorporation events in a multiplex manner. In embodiments using surface-bound target nucleic acids, the target nucleic acids can be in an array format. In an array format, the target nucleic acids can be typically bound to a surface in a spatially distinguishable manner. The target nucleic acids can be bound by direct covalent attachment, attachment to a bead or other particle or binding to a polymerase or other molecule that is attached to the surface. The array can include a single copy of a target nucleic acid at each site (also referred to as a feature) or multiple copies having the same sequence can be present at each site or feature. Multiple copies can be produced by amplification methods such as, bridge amplification or emulsion PCR as described in further detail below.

The methods set forth herein can use arrays having features at any of a variety of densities including, for example, at least about 10 features/cm2, 100 features/cm2, 500 features/cm2, 1,000 features/cm2, 5,000 features/cm2, 10,000 features/cm2, 50,000 features/cm2, 100,000 features/cm2, 1,000,000 features/cm2, 5,000,000 features/cm2, or higher.

An advantage of the methods set forth herein is that they provide for rapid and efficient detection of a plurality of target nucleic acid in parallel. Accordingly the present disclosure provides integrated systems capable of preparing and detecting nucleic acids using techniques known in the art such as those exemplified above. Thus, an integrated system of the present disclosure can include fluidic components capable of delivering amplification reagents and/or sequencing reagents to one or more immobilized DNA fragments, the system comprising components such as pumps, valves, reservoirs, fluidic lines and the like. A flow cell can be configured and/or used in an integrated system for detection of target nucleic acids. Exemplary flow cells are described, for example, in US 2010/0111768 A1 and US Ser. No. 13/273,666. As exemplified for flow cells, one or more of the fluidic components of an integrated system can be used for an amplification method and for a detection method. Taking a nucleic acid sequencing embodiment as an example, one or more of the fluidic components of an integrated system can be used for an amplification method set forth herein and for the delivery of sequencing reagents in a sequencing method such as those exemplified above. Alternatively, an integrated system can include separate fluidic systems to carry out amplification methods and to carry out detection methods. Examples of integrated sequencing systems that are capable of creating amplified nucleic acids and also determining the sequence of the nucleic acids include, without limitation, the MiSeqTM platform (Illumina, Inc., San Diego, CA) and devices described in US Ser. No. 13/273,666.

The sequencing system described above sequences nucleic acid polymers present in samples received by a sequencing device. As defined herein, "sample" and its derivatives, is used in its broadest sense and includes any specimen, culture and the like that is suspected of including a target. In some embodiments, the sample comprises DNA, RNA, PNA, LNA, chimeric or hybrid forms of nucleic acids. The sample can include any biological, clinical, surgical, agricultural, atmospheric or aquatic-based specimen containing one or more nucleic acids. The term also includes any isolated nucleic acid sample such a genomic DNA, fresh-frozen or formalin-fixed paraffin-embedded nucleic acid specimen. It is also envisioned that the sample can be from a single individual, a collection of nucleic acid samples from genetically related members, nucleic acid samples from genetically unrelated members, nucleic acid samples (matched) from a single individual such as a tumor sample and normal tissue sample, or sample from a single source that contains two distinct forms of genetic material such as maternal and fetal DNA obtained from a maternal subject, or the presence of contaminating bacterial DNA in a sample that contains plant or animal DNA. In some embodiments, the source of nucleic acid material can include nucleic acids obtained from a newborn, for example as typically used for newborn screening.

The nucleic acid sample can include high molecular weight material such as genomic DNA (gDNA). The sample can include low molecular weight material such as nucleic acid molecules obtained from FFPE or archived DNA samples. In another embodiment, low molecular weight material includes enzymatically or mechanically fragmented DNA. The sample can include cell-free circulating DNA. In some embodiments, the sample can include nucleic acid molecules obtained from biopsies, tumors, scrapings, swabs, blood, mucus, urine, plasma, semen, hair, laser capture micro-dissections, surgical resections, and other clinical or laboratory obtained samples. In some embodiments, the sample can be an epidemiological, agricultural, forensic or pathogenic sample. In some embodiments, the sample can include nucleic acid molecules obtained from an animal such as a human or mammalian source. In another embodiment, the sample can include nucleic acid molecules obtained from a non-mammalian source such as a plant, bacteria, virus or fungus. In some embodiments, the source of the nucleic acid molecules may be an archived or extinct sample or species.

Further, the methods and compositions disclosed herein may be useful to amplify a nucleic acid sample having low-quality nucleic acid molecules, such as degraded and/or fragmented genomic DNA from a forensic sample. In one embodiment, forensic samples can include nucleic acids obtained from a crime scene, nucleic acids obtained from a missing persons DNA database, nucleic acids obtained from a laboratory associated with a forensic investigation or include forensic samples obtained by law enforcement agencies, one or more military services or any such personnel. The nucleic acid sample may be a purified sample or a crude DNA containing lysate, for example derived from a buccal swab, paper, fabric or other substrate that may be impregnated with saliva, blood, or other bodily fluids. As such, in some embodiments, the nucleic acid sample may comprise low amounts of, or fragmented portions of DNA, such as genomic DNA. In some embodiments, target sequences can be present in one or more bodily fluids including but not limited to, blood, sputum, plasma, semen, urine and serum. In some embodiments, target sequences can be obtained from hair, skin, tissue samples, autopsy or remains of a victim. In some embodiments, nucleic acids including one or more target sequences can be obtained from a deceased animal or human. In some embodiments, target sequences can include nucleic acids obtained from non-human DNA such a microbial, plant or entomological DNA. In some embodiments, target sequences or amplified target sequences are directed to purposes of human identification. In some embodiments, the disclosure relates generally to methods for identifying characteristics of a forensic sample. In some embodiments, the disclosure relates generally to human identification methods using one or more target specific primers disclosed herein or one or more target specific primers designed using the primer design criteria outlined herein. In one embodiment, a forensic or human identification sample containing at least one target sequence can be amplified using any one or more of the target-specific primers disclosed herein or using the primer criteria outlined herein.

The components of the diagnostic workflow system 106 can include software, hardware, or both. For example, the components of the diagnostic workflow system 106 can include one or more instructions stored on a computer-readable storage medium and executable by processors of one or more computing devices (e.g., the client device 108). When executed by the one or more processors, the computer-executable instructions of the diagnostic workflow system 106 can cause the computing devices to perform the bubble detection methods described herein. Alternatively, the components of the diagnostic workflow system 106 can comprise hardware, such as special purpose processing devices to perform a certain function or group of functions. Additionally, or alternatively, the components of the diagnostic workflow system 106 can include a combination of computer-executable instructions and hardware.

Furthermore, the components of the diagnostic workflow system 106 performing the functions described herein with respect to the diagnostic workflow system 106 may, for example, be implemented as part of a stand-alone application, as a module of an application, as a plug-in for applications, as a library function or functions that may be called by other applications, and/or as a cloud-computing model. Thus, components of the diagnostic workflow system 106 may be implemented as part of a stand-alone application on a personal computing device or a mobile device. Additionally, or alternatively, the components of the diagnostic workflow system 106 may be implemented in any application that provides sequencing services including, but not limited to Illumina BaseSpace, Illumina DRAGEN, or Illumina TruSight software. "Illumina," "BaseSpace," "DRAGEN," and "TruSight," are either registered trademarks or trademarks of Illumina, Inc. in the United States and/or other countries.

Embodiments of the present disclosure may comprise or utilize a special purpose or general-purpose computer including computer hardware, such as, for example, one or more processors and system memory, as discussed in greater detail below. Embodiments within the scope of the present disclosure also include physical and other computer-readable media for carrying or storing computer-executable instructions and/or data structures. In particular, one or more of the processes described herein may be implemented at least in part as instructions embodied in a non-transitory computer-readable medium and executable by one or more computing devices (e.g., any of the media content access devices described herein). In general, a processor (e.g., a microprocessor) receives instructions, from a non-transitory computer-readable medium, (e.g., a memory, etc.), and executes those instructions, thereby performing one or more processes, including one or more of the processes described herein.

Computer-readable media can be any available media that can be accessed by a general purpose or special purpose computer system. Computer-readable media that store computer-executable instructions are non-transitory computer-readable storage media (devices). Computer-readable media that carry computer-executable instructions are transmission media. Thus, by way of example, and not limitation, embodiments of the disclosure can comprise at least two distinctly different kinds of computer-readable media: non-transitory computer-readable storage media (devices) and transmission media.

Non-transitory computer-readable storage media (devices) includes RAM, ROM, EEPROM, CD-ROM, solid state drives (SSDs) (e.g., based on RAM), Flash memory, phase-change memory (PCM), other types of memory, other optical disk storage, magnetic disk storage or other magnetic storage devices, or any other medium which can be used to store desired program code means in the form of computer-executable instructions or data structures and which can be accessed by a general purpose or special purpose computer.

A "network" is defined as one or more data links that enable the transport of electronic data between computer systems and/or modules and/or other electronic devices. When information is transferred or provided over a network or another communications connection (either hardwired, wireless, or a combination of hardwired or wireless) to a computer, the computer properly views the connection as a transmission medium. Transmissions media can include a network and/or data links which can be used to carry desired program code means in the form of computer-executable instructions or data structures and which can be accessed by a general purpose or special purpose computer. Combinations of the above should also be included within the scope of computer-readable media.

Further, upon reaching various computer system components, program code means in the form of computer-executable instructions or data structures can be transferred automatically from transmission media to non-transitory computer-readable storage media (devices) (or vice versa). For example, computer-executable instructions or data structures received over a network or data link can be buffered in RAM within a network interface module (e.g., a NIC), and then eventually transferred to computer system RAM and/or to less volatile computer storage media (devices) at a computer system. Thus, it should be understood that non-transitory computer-readable storage media (devices) can be included in computer system components that also (or even primarily) utilize transmission media.

Computer-executable instructions comprise, for example, instructions and data which, when executed at a processor, cause a general purpose computer, special purpose computer, or special purpose processing device to perform a certain function or group of functions. In some embodiments, computer-executable instructions are executed on a general-purpose computer to turn the general-purpose computer into a special purpose computer implementing elements of the disclosure. The computer executable instructions may be, for example, binaries, intermediate format instructions such as assembly language, or even source code. Although the subject matter has been described in language specific to structural features and/or methodological acts, it is to be understood that the subject matter defined in the appended claims is not necessarily limited to the described features or acts described above. Rather, the described features and acts are disclosed as example forms of implementing the claims.

Those skilled in the art will appreciate that the disclosure may be practiced in network computing environments with many types of computer system configurations, including, personal computers, desktop computers, laptop computers, message processors, hand-held devices, multiprocessor systems, microprocessor-based or programmable consumer electronics, network PCs, minicomputers, mainframe computers, mobile telephones, PDAs, tablets, pagers, routers, switches, and the like. The disclosure may also be practiced in distributed system environments where local and remote computer systems, which are linked (either by hardwired data links, wireless data links, or by a combination of hardwired and wireless data links) through a network, both perform tasks. In a distributed system environment, program modules may be located in both local and remote memory storage devices.

Embodiments of the present disclosure can also be implemented in cloud computing environments. In this description, "cloud computing" is defined as a model for enabling on-demand network access to a shared pool of configurable computing resources. For example, cloud computing can be employed in the marketplace to offer ubiquitous and convenient on-demand access to the shared pool of configurable computing resources. The shared pool of configurable computing resources can be rapidly provisioned via virtualization and released with low management effort or service provider interaction, and then scaled accordingly.

A cloud-computing model can be composed of various characteristics such as, for example, on-demand self-service, broad network access, resource pooling, rapid elasticity, measured service, and so forth. A cloud-computing model can also expose various service models, such as, for example, Software as a Service (SaaS), Platform as a Service (PaaS), and Infrastructure as a Service (IaaS). A cloud-computing model can also be deployed using different deployment models such as private cloud, community cloud, public cloud, hybrid cloud, and so forth. In this description and in the claims, a "cloud-computing environment" is an environment in which cloud computing is employed.

FIG. 9 illustrates a block diagram of a computing device 900 that may be configured to perform one or more of the processes described above. One will appreciate that one or more computing devices such as the computing device 900 may implement the diagnostic workflow system 106 and the genomic analysis platform 104. As shown by FIG. 9, the computing device 900 can comprise a processor 902, a memory 904, a storage device 906, an I/O interface 908, and a communication interface 910, which may be communicatively coupled by way of a communication infrastructure 912. In certain embodiments, the computing device 900 can include fewer or more components than those shown in FIG. 9. The following paragraphs describe components of the computing device 900 shown in FIG. 9 in additional detail.

In one or more embodiments, the processor 902 includes hardware for executing instructions, such as those making up a computer program. As an example, and not by way of limitation, to execute instructions for dynamically modifying workflows, the processor 902 may retrieve (or fetch) the instructions from an internal register, an internal cache, the memory 904, or the storage device 906 and decode and execute them. The memory 904 may be a volatile or nonvolatile memory used for storing data, metadata, and programs for execution by the processor(s). The storage device 906 includes storage, such as a hard disk, flash disk drive, or other digital storage device, for storing data or instructions for performing the methods described herein.

The I/O interface 908 allows a user to provide input to, receive output from, and otherwise transfer data to and receive data from computing device 900. The I/O interface 908 may include a mouse, a keypad or a keyboard, a touch screen, a camera, an optical scanner, network interface, modem, other known I/O devices or a combination of such I/O interfaces. The I/O interface 908 may include one or more devices for presenting output to a user, including, but not limited to, a graphics engine, a display (e.g., a display screen), one or more output drivers (e.g., display drivers), one or more audio speakers, and one or more audio drivers. In certain embodiments, the I/O interface 908 is configured to provide graphical data to a display for presentation to a user. The graphical data may be representative of one or more graphical user interfaces and/or any other graphical content as may serve a particular implementation.

The communication interface 910 can include hardware, software, or both. In any event, the communication interface 910 can provide one or more interfaces for communication (such as, for example, packet-based communication) between the computing device 900 and one or more other computing devices or networks. As an example, and not by way of limitation, the communication interface 910 may include a network interface controller (NIC) or network adapter for communicating with an Ethernet or other wire-based network or a wireless NIC (WNIC) or wireless adapter for communicating with a wireless network, such as a WI-FI.

Additionally, the communication interface 910 may facilitate communications with various types of wired or wireless networks. The communication interface 910 may also facilitate communications using various communication protocols. The communication infrastructure 912 may also include hardware, software, or both that couples components of the computing device 900 to each other. For example, the communication interface 910 may use one or more networks and/or protocols to enable a plurality of computing devices connected by a particular infrastructure to communicate with each other to perform one or more aspects of the processes described herein. To illustrate, the sequencing process can allow a plurality of devices (e.g., a client device, sequencing device, and server device(s)) to exchange information such as sequencing data and error notifications.

In the foregoing specification, the present disclosure has been described with reference to specific exemplary embodiments thereof. Various embodiments and aspects of the present disclosure(s) are described with reference to details discussed herein, and the accompanying drawings illustrate the various embodiments. The description above and drawings are illustrative of the disclosure and are not to be construed as limiting the disclosure. Numerous specific details are described to provide a thorough understanding of various embodiments of the present disclosure.

The present disclosure may be embodied in other specific forms. The described embodiments are to be considered in all respects only as illustrative and not restrictive. For example, the methods described herein may be performed with less or more steps/acts or the steps/acts may be performed in differing orders. Additionally, the steps/acts described herein may be repeated or performed in parallel with one another or in parallel with different instances of the same or similar steps/acts. The scope of the present application is, therefore, indicated by the appended claims rather than by the foregoing description.

As used herein, the term "object" includes all things that are suitable for imaging, viewing, analyzing, inspecting, or profiling with the optical systems described herein. By way of example only, objects may include semiconductor wafers or chips, recordable media, samples, flow cells, microparticles, slides, or microarrays. Objects generally include one or more surfaces and/or one or more interfaces that a user may desire to image, view, analyze, inspect, and/or determine a profile thereof. The objects may have surfaces or interfaces with relief features such as wells, pits, ridges, bumps, beads or the like.

As indicated above in the description of a "sample," a sample may be imaged or scanned for subsequent analysis. In particular embodiments, a sample may include biological or chemical substances of interests and, optionally, an optical substrate that supports the biological or chemical substances. As such, a sample may or may not include an optical substrate. As used herein, the term "biological or chemical substances" is not intended to be limiting, but may include a variety of biological or chemical substances that are suitable for being imaged or examined with the optical systems described herein. For example, biological or chemical substances include biomolecules, such as nucleosides, nucleic acids, polynucleotides, oligonucleotides, proteins, enzymes, polypeptides, antibodies, antigens, ligands, receptors, polysaccharide, carbohydrate, polyphosphates, nanopores, organelles, lipid layers, cells, tissues, organisms, and biologically active chemical compound(s) such as analogs or mimetics of the aforementioned species.

The biological or chemical substances may be supported by an optical substrate. As used herein, the term "optical substrate" is not intended to be limiting, but may include various materials that support the biological or chemical substances and permit the biological or chemical substances to be at least one of viewed, imaged, and examined. For example, the optical substrate may comprise a transparent material that reflects a portion of incident light and refracts a portion of the incident light. Alternatively, the optical substrate may be, for example, a mirror that reflects the incident light entirely such that no light is transmitted through the optical substrate. Typically, the optical substrate has a flat surface. However, the optical substrate can have a surface with relief features such as wells, pits, ridges, bumps, beads or the like.

In an exemplary embodiment, the optical substrate is a flow cell having flow channels where nucleic acids are sequenced. However, in alternative embodiments, the optical substrate may include one or more slides, planar chips (such as those used in microarrays), or microparticles. In such cases where the optical substrate includes a plurality of microparticles that support the biological or chemical substances, the microparticles may be held by another optical substrate, such as a slide or grooved plate. In particular embodiments, the optical substrate includes diffraction grating based encoded optical identification elements similar to or the same as those described in pending U.S. patent application Ser. No. 10/661,234, entitled Diffraction Grating Based Optical Identification Element, filed Sep. 12, 2003, discussed more hereinafter. A bead cell or plate for holding the optical identification elements may be similar to or the same as that described in pending U.S. patent application Ser. No. 10/661,836, entitled "Method and Apparatus for Aligning Microbeads in Order to Interrogate the Same", filed Sep. 12, 2003, and U.S. Pat. No. 7,164,533, entitled "Hybrid Random Bead/Chip Based Microarray", issued Jan. 16, 2007, as well as U.S. patent application Ser. No. 60/609,583, entitled "Improved Method and Apparatus for Aligning Microbeads in Order to Interrogate the Same", filed Sep. 13, 2004, Ser. No. 60/1010,910, entitled "Method and Apparatus for Aligning Microbeads in Order to Interrogate the Same", filed Sep. 17, 2004.

As used herein, the term "optical components" or "focus components" includes various elements that affect the transmission of light. Optical components may be, for example, reflectors, dichroics, beam splitters, collimators, lenses, filters, wedges, prisms, mirrors, and the like.

By way of example, optical systems described herein may be constructed to include various components and assemblies as described in PCT application PCT/US07/07991, entitled "System and Devices for Sequence by Synthesis Analysis", filed Mar. 30, 2007 and/or to include various components and assemblies as described in PCT application PCT/US2008/077850, entitled "Fluorescence Excitation and Detection System and Method", filed Sep. 26, 2008. In particular embodiments, optical systems can include various components and assemblies as described in U.S. Pat. No.7,329,860. Optical systems can also include various components and assemblies as described in U.S. patent application Ser. No.12/638,770, filed on Dec. 15, 2009.

In particular embodiments, methods, and optical systems described herein may be used for sequencing nucleic acids. For example, sequencing-by-synthesis (SBS) protocols are particularly applicable. In SBS, a plurality of fluorescently labeled modified nucleotides are used to sequence dense clusters of amplified DNA (possibly millions of clusters) present on the surface of an optical substrate (e.g., a surface that at least partially defines a channel in a flow cell). The flow cells may contain nucleic acid samples for sequencing where the flow cells are placed within the appropriate flow cell holders. The samples for sequencing can take the form of single nucleic acid molecules that are separated from each other so as to be individually resolvable, amplified populations of a nucleic acid molecules in the form of clusters or other features, or beads that are attached to one or more molecules of nucleic acid. The nucleic acids can be prepared such that they comprise an oligonucleotide primer adjacent to an unknown target sequence. To initiate the first SBS sequencing cycle, one or more differently labeled nucleotides, and DNA polymerase, etc., can be flowed into/through the flow cell by a fluid flow subsystem (not shown). Either a single type of nucleotide can be added at a time, or the nucleotides used in the sequencing procedure can be specially designed to possess a reversible termination property, thus allowing each cycle of the sequencing reaction to occur simultaneously in the presence of several types of labeled nucleotides (e.g., A, C, T, G). The nucleotides can include detectable label moieties such as fluorophores. Where the four nucleotides are mixed together, the polymerase is able to select the correct base to incorporate and each sequence is extended by a single base. One or more lasers may excite the nucleic acids and induce fluorescence. The fluorescence emitted from the nucleic acids is based upon the fluorophores of the incorporated base, and different fluorophores may emit different wavelengths of emission light. Exemplary sequencing methods are described, for example, in Bentley et al., Nature 456:53-59 (2008), WO 04/018497; U.S. Pat. No. 7,057,026; WO 91/06678; WO 07/123,744; U.S. Pat. No. 7,329,492; U.S. Pat. No. 7,211,414; U.S. Pat. No. 7,315,019; U.S. Pat. No. 7,405,281, and US 2008/0108082.

Other sequencing techniques that are applicable for use of the methods and systems set forth herein are pyrosequencing, nanopore sequencing, and sequencing by ligation. Exemplary pyrosequencing techniques and samples that are particularly useful are described in U.S. Pat. No. 6,210,891; U.S. Pat. No. 6,258,568; U.S. Pat. No. 6,274,320 and Ronaghi, Genome Research 11:3-11 (2001). Exemplary nanopore techniques and samples that are also useful are described in Deamer et al., Acc. Chem. Res. 35:817-825 (2002); Li et al., Nat. Mater. 2:611-615 (2003); Soni et al., Clin Chem. 53:1996-2001 (2007) Healy et al., Nanomed 2:459-481 (2007) and Cockroft et al., J. am. Chem. Soc. 130:818-820; and U.S. Pat. No. 7,001,792. Any of a variety of samples can be used in these systems such as substrates having beads generated by emulsion PCR, substrates having zero-mode waveguides, substrates having biological nanopores in lipid bilayers, solid-state substrates having synthetic nanopores, and others known in the art. Such samples are described in the context of various sequencing techniques in the references cited above and further in US 2005/0042648; US 2005/0079510; US 2005/0130173; and WO 05/010145.

In other embodiments, optical systems described herein may be utilized for detection of samples that include microarrays. A microarray may include a population of different probe molecules that are attached to one or more substrates such that the different probe molecules can be differentiated from each other according to relative location. An array can include different probe molecules, or populations of the probe molecules, that are each located at a different addressable location on a substrate. Alternatively, a microarray can include separate optical substrates, such as beads, each bearing a different probe molecule, or population of the probe molecules, that can be identified according to the locations of the optical substrates on a surface to which the substrates are attached or according to the locations of the substrates in a liquid. Exemplary arrays in which separate substrates are located on a surface include, without limitation, a Sentrix^{®} Array or Sentrix^{®} BeadChip Array available from Illumina^{®}, Inc. (San Diego, Calif.) or others including beads in wells such as those described in U.S. Pat. Nos. 6,266,459, 6,355,431, 6,770,441, and 6,859,570; and PCT Publication No. WO 00/63437. Other arrays having particles on a surface include those set forth in US 2005/0227252; WO 05/033681; and WO 04/024328.

Any of a variety of microarrays known in the art, including, for example, those set forth herein, can be used in embodiments of the invention. A typical microarray contains sites, sometimes referred to as features, each having a population of probes. The population of probes at each site is typically homogenous having a single species of probe, but in some embodiments the populations can each be heterogeneous. Sites or features of an array are typically discrete, being separated with spaces between each other. The size of the probe sites and/or spacing between the sites can vary such that arrays can be high density, medium density or lower density. High density arrays are characterized as having sites separated by less than about 15 µm. Medium density arrays have sites separated by about 15 to 30 µm, while low density arrays have sites separated by greater than 30 µm. An array useful in the invention can have sites that are separated by less than 100 µm, 50 µm, 10 µm, 5 µm, 1 µm, or 0.5 µm. An apparatus or method of an embodiment of the invention can be used to image an array at a resolution sufficient to distinguish sites at the above densities or density ranges.

Further examples of commercially available microarrays that can be used include, for example, an Affymetrix^{®} GeneChip^{®} microarray or other microarray synthesized in accordance with techniques sometimes referred to as VLSIPS^{™} (Very Large Scale Immobilized Polymer Synthesis) technologies as described, for example, in U.S. Pat. Nos. 5,324,633; 5,744,305; 5,451,683; 5,482,867; 5,491,074; 5,624,711; 5,795,716; 5,831,070; 5,856,101; 5,858,659; 5,874,219; 5,968,740; 5,974,164; 5,981,185; 5,981,956; 6,025,601; 6,033,860; 6,090,555; 6,136,269; 6,022,963; 6,083,697; 6,291,183; 6,309,831; 6,416,949; 6,428,752 and 6,482,591. A spotted microarray can also be used in a method according to an embodiment of the invention. An exemplary spotted microarray is a CodeLink^{™} Array available from Amersham Biosciences. Another microarray that is useful is one that is manufactured using inkjet printing methods such as SurePrint^{™} Technology available from Agilent Technologies.

The systems and methods set forth herein can be used to detect the presence of a particular target molecule in a sample contacted with the microarray. This can be determined, for example, based on binding of a labeled target analyte to a particular probe of the microarray or due to a target-dependent modification of a particular probe to incorporate, remove, or alter a label at the probe location. Any one of several assays can be used to identify or characterize targets using a microarray as described, for example, in U. S. Patent Application Publication Nos. 2003/0108867; 2003/0108900; 2003/0170684; 2003/0207295; or 2005/0181394.

Exemplary labels that can be detected in accordance with embodiments of the invention, for example, when present on a microarray include, but are not limited to, a chromophore; luminophore; fluorophore; optically encoded nanoparticles; particles encoded with a diffraction-grating; electrochemiluminescent label such as Ru(bpy)³²⁺; or moiety that can be detected based on an optical characteristic. Fluorophores that may be useful include, for example, fluorescent lanthanide complexes, including those of Europium and Terbium, fluorescein, rhodamine, tetramethylrhodamine, eosin, erythrosin, coumarin, methyl-coumarins, pyrene, Malacite green, Cy3, Cy5, stilbene, Lucifer Yellow, Cascade Blue^{™}, Texas Red, alexa dyes, phycoerythin, bodipy, and others known in the art such as those described in Haugland, Molecular Probes Handbook, (Eugene, Oreg.) 6th Edition; The Synthegen catalog (Houston, Tex.), Lakowicz, Principles of Fluorescence Spectroscopy, 2nd Ed., Plenum Press New York (1999), or WO 98/59066.

In particular embodiments, the optical system can be configured for Time Delay Integration (TDI) for example in line scanning embodiments as described, for example, in U.S. Pat. No. 7,329,860. By way of example, the optical assembly may have a 0.75 NA lens and a focus accuracy of +/-125 to 500 nm. The resolution can be 50 to 100 nm. The system may be able to obtain 1,000-10,000 measurements/second unfiltered.

Although embodiments are exemplified with regard to detection of samples that includes biological or chemical substances supported by an optical substrate, it will be understood that other samples can be analyzed, examined, or imaged by the embodiments described herein. Other exemplary samples include, but are not limited to, biological specimens such as cells or tissues, electronic chips such as those used in computer processors, or the like. Examples of some of the applications include microscopy, satellite scanners, high-resolution reprographics, fluorescent image acquisition, analyzing and sequencing of nucleic acids, DNA sequencing, sequencing-by-synthesis, imaging of microarrays, imaging of holographically encoded microparticles and the like.

In other embodiments, the optical systems may be configured to inspect an object to determine certain features or structures of the object. For example, the optical systems may be used to inspect a surface of the object, (e.g., semiconductor chip, silicon wafer) to determine whether there are any deviations or defects on the surface.

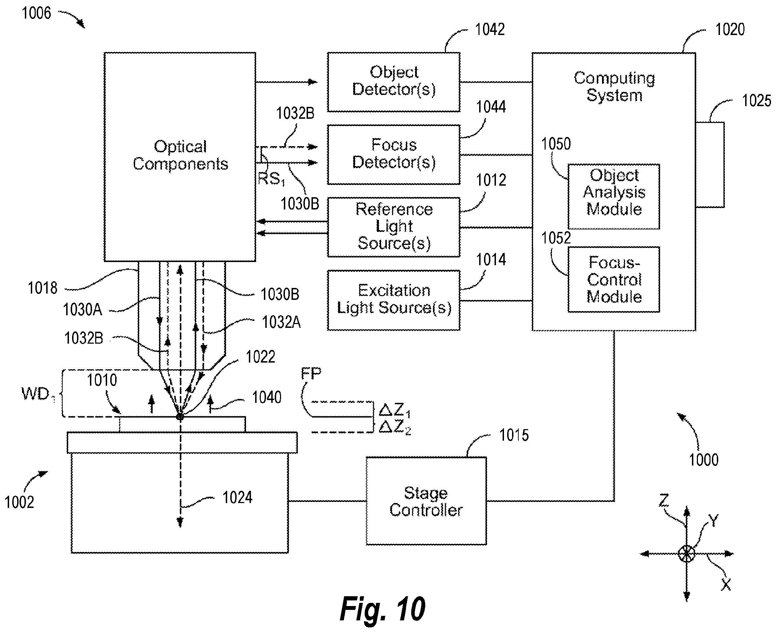

FIG. 10 illustrates a block diagram of an optical system 1000 formed in accordance with one embodiment. By way of example only, the optical system 1000 may be a sampler imager that images a sample of interest for analysis. In other embodiments, the optical system 1000 may be a profilometer that determines a surface profile (e.g., topography) of an object. Furthermore, various other types of optical systems may use the mechanisms and systems described herein. In the illustrated embodiment, the optical system 1000 includes an optical assembly 1006, an object holder 1002 for supporting an object 1010 near a focal plane FP of the optical assembly 1006, and a stage controller 1015 that is configured to move the object holder 1002 in a lateral direction (along an X-axis and/or a Y-axis that extend into the page) or in a vertical/elevational direction along a Z-axis. The optical system 1000 may also include a system controller or computing system 1020 that is operatively coupled to the optical assembly 1006, the stage controller 1015, and/or the object holder 1002.

In particular embodiments, the optical system 1000 is a sample imager configured to image samples. Although not shown, a sample imager may include other sub-systems or devices for performing various assay protocols. By way of example only, the sample may include a flow cell having flow channels. The sample imager may include a fluid control system that includes liquid reservoirs that are fluidically coupled to the flow channels through a fluidic network. The sample imager may also include a temperature control system that may have a heater/cooler configured to regulate a temperature of the sample and/or the fluid that flows through the sample. The temperature control system may include sensors that detect a temperature of the fluids.

As shown, the optical assembly 1006 is configured to direct input light to an object 1010 and receive and direct output light to one or more detectors. The output light may be input light that was at least one of reflected and refracted by the object 1010 and/or the output light may be light emitted from the object 1010. To direct the input light, the optical assembly 1006 may include at least one reference light source 1012 and at least one excitation light source 1014 that direct light, such as light beams having predetermined wavelengths, through one or more optical components of the optical assembly 1006. The optical assembly 1006 may include various optical components, including a conjugate lens 1018, for directing the input light toward the object 1010 and directing the output light toward the detector(s).

In the exemplary embodiment, the reference light source 1012 may be used by a distance measuring system or a focus-control system (or focusing mechanism) of the optical system 1000 and the excitation light source 1014 may be used to excite the biological or chemical substances of the object 1010 when the object 1010 includes a biological or chemical sample. The excitation light source 1014 may be arranged to illuminate a bottom surface of the object 1010, such as in TIRF imaging, or may be arranged to illuminate a top surface of the object 1010, such as in epi-fluorescent imaging. As shown in FIG. 10, the conjugate lens 1018 directs the input light to a focal region 1022 lying within the focal plane FP. The lens 1018 has an optical axis 1024 and is positioned a working distance WD₁ away from the object 1010 measured along the optical axis 1024. The stage controller 1015 may move the object 1010 in the Z-direction to adjust the working distance WD₁ so that, for example, a portion of the object 1010 is within the focal region 1022.

To determine whether the object 1010 is in focus (i.e., sufficiently within the focal region 1022 or the focal plane FP), the optical assembly 1006 is configured to direct at least one pair of light beams to the focal region 1022 where the object 1010 is approximately located. The object 1010 reflects the light beams. More specifically, an exterior surface of the object 1010 or an interface within the object 1010 reflects the light beams. The reflected light beams then return to and propagate through the lens 1018. As shown, each light beam has an optical path that includes a portion that has not yet been reflected by the object 1010 and a portion that has been reflected by the object 1010. The portions of the optical paths prior to reflection are designated as incident light beams 1030A and 1032A and are indicated with arrows pointing toward the object 1010. The portions of the optical paths that have been reflected by the object 1010 are designated as reflected light beams 1030B and 1032B and are indicated with arrows pointing away from the object 1010. For illustrative purposes, the light beams 1030A, 1030B, 1032A, and 1032B are shown as having different optical paths within the lens 1018 and near the object 1010. However, in the exemplary embodiment, the light beams 1030A and 1032B propagate in opposite directions and are configured to have the same or substantially overlapping optical paths within the lens 1018 and near the object 1010, and the light beams 1030B and 1032A propagate in opposite directions and are configured to have the same or substantially overlapping optical paths within the lens 1018 and near the object 1010.

In the embodiment shown in FIG. 10, light beams 1030A, 1030B, 1032A, and 1032B pass through the same lens that is used for imaging. In an alternative embodiment, the light beams used for distance measurement or focus determination can pass through a different lens that is not used for imaging. In this alternative embodiment, the lens 1018 is dedicated to passing beams 1030A, 1030B, 1032A, and 1032B for distance measurement or focus determination, and a separate lens (not shown) is used for imaging the object 1010. Similarly, it will be understood that the systems and methods set forth herein for focus determination and distance measurement can occur using a common objective lens that is shared with the imaging optics or, alternatively, the objective lenses exemplified herein can be dedicated to focus determination or distance measurement.

The reflected light beams 1030B and 1032B propagate through the lens 1018 and may, optionally, be further directed by other optical components of the optical assembly 1006. As shown, the reflected light beams 1030B and 1032B are detected by at least one focus detector 1044. In the illustrated embodiment, both reflected light beams 1030B and 1032B are detected by a single focus detector 1044. The reflected light beams may be used to determine relative separation RS₁. For example, the relative separation RS₁ may be determined by the distance separating the beam spots from the impinging reflected light beams 1030B and 1032B on the focus detector 1044 (i.e., a separation distance). The relative separation RS₁ may be used to determine a degree-of-focus of the optical system 1000 with respect to the object 1010. However, in alternative embodiments, each reflected light beam 1030B and 1032B may be detected by a separate corresponding focus detector 1044 and the relative separation RS₁ may be determined based upon a location of the beam spots on the corresponding focus detectors 1044.

If the object 1010 is not within a sufficient degree-of-focus, the computing system 1020 may operate the stage controller 1015 to move the object holder 1002 to a desired position. Alternatively or in addition to moving the object holder 1002, the optical assembly 1006 may be moved in the Z-direction and/or along the XY plane.

For example, the object 1010 may be relatively moved a distance ΔZ₁ toward the focal plane FP if the object 1010 is located above the focal plane FP (or focal region 1022), or the object 1010 may be relatively moved a distance ΔZ₂ toward the focal plane FP if the object 1010 is located below the focal plane FP (or focal region 1022). In some embodiments, the optical system 1000 may substitute the lens 1018 with another lens 1018 or other optical components to move the focal region 1022 of the optical assembly 1006.

The example set forth above and in FIG. 10 has been presented with respect to a system for controlling focus or for determining degree-of-focus. The system is also useful for determining the working distance WD₁ between the object 1010 and the lens 1018. In such embodiments, the focus detector 1044 can function as a working distance detector and the distance separating the beam spots on the working distance detector can be used to determine the working distance between the object 1010 and the lens 1018. For ease of description, various embodiments of the systems and methods are exemplified herein with regard to controlling focus or determining degree-of-focus. It will be understood that the systems and methods can also be used to determine the working distance between an object and a lens. Likewise, the systems and methods may also be used to determine a surface profile of an object.

In the exemplary embodiment, during operation, the excitation light source 1014 directs input light (not shown) onto the object 1010 to excite fluorescently-labeled biological or chemical substances. The labels of the biological or chemical substances provide light signals 1040 (also called light emissions) having predetermined wavelength(s). The light signals 1040 are received by the lens 1018 and then directed by other optical components of the optical assembly 1006 to at least one object detector 1042. Although the illustrated embodiment only shows one object detector 1042, the object detector 1042 may comprise multiple detectors. For example, the object detector 1042 may include a first detector configured to detect one or more wavelengths of light and a second detector configured to detect one or more different wavelengths of light. The optical assembly 1006 may include a lens/filter assembly that directs different light signals along different optical paths toward the corresponding object detectors. Such optical systems are described in further detail by PCT Application No. PCT/US07/07991, entitled "System and Devices for Sequence by Synthesis Analysis", filed Mar. 30, 2007 and PCT Application No. PCT/US2008/077850, entitled "Fluorescence Excitation and Detection System and Method", filed Sep. 26, 2008.

The object detector 1042 communicates object data relating to the detected light signals 1040 to the computing system 1020. The computing system 1020 may then record, process, analyze, and/or communicate the data to other users or computing systems, including remote computing systems through a communication line (e.g., Internet). By way of example, the object data may include imaging data that is processed to generate an image(s) of the object 1010. The images may then be analyzed by the computing system and/or a user of the optical system 1000. In other embodiments, the object data may not only include light emissions from the biological or chemical substances, but may also include light that is at least one of reflected and refracted by the optical substrate or other components. For example, the light signals 1040 may include light that has been reflected by encoded microparticles, such as the holographically encoded optical identification elements described above.

In some embodiments, a single detector may provide both functions as described above with respect to the object and focus detectors 1042 and 1044. For example, a single detector may detect the reflected light beams 1030B and 1032B and also the light signals 1040.

The optical system 1000 may include a user interface 1025 that interacts with the user through the computing system 1020. For example, the user interface 1025 may include a display (not shown) that shows and requests information from a user and a user input device (not shown) to receive user inputs.

The computing system 1020 may include, among other things, an object analysis module 1050 and a focus-control module 1052. The focus-control module 1052 is configured to receive focus data obtained by the focus detector 1044. The focus data may include signals representative of the beam spots incident upon the focus detector 1044. The data may be processed to determine relative separation (e.g., separation distance between the beam spots). A degree-of-focus of the optical system 1000 with respect to the object 1010 may then be determined based upon the relative separation. In particular embodiments, the working distance WD₁ between the object 1010 and lens 1018 can be determined. Likewise, the object analysis module 1050 may receive object data obtained by the object detectors 1042. The object analysis module may process or analyze the object data to generate images of the object.

Furthermore, the computing system 1020 may include any processor-based or microprocessor-based system, including systems using microcontrollers, reduced instruction set computers (RISC), application specific integrated circuits (ASICs), field programmable gate array (FPGAs), logic circuits, and any other circuit or processor capable of executing functions described herein. The above examples are exemplary only, and are thus not intended to limit in any way the definition and/or meaning of the term system controller. In the exemplary embodiment, the computing system 1020 executes a set of instructions that are stored in one or more storage elements, memories, or modules in order to at least one of obtain and analyze object data. Storage elements may be in the form of information sources or physical memory elements within the optical system 1000.

The set of instructions may include various commands that instruct the optical system 1000 to perform specific protocols. For example, the set of instructions may include various commands for performing assays and imaging the object 1010 or for determining a surface profile of the object 1010. The set of instructions may be in the form of a software program. As used herein, the terms "software" and "firmware" are interchangeable, and include any computer program stored in memory for execution by a computer, including RAM memory, ROM memory, EPROM memory, EEPROM memory, and non-volatile RAM (NVRAM) memory. The above memory types are exemplary only, and are thus not limiting as to the types of memory usable for storage of a computer program.

As described above, the excitation light source 1014 generates an excitation light that is directed onto the object 1010. The excitation light source 1014 may generate one or more laser beams at one or more predetermined excitation wavelengths. The light may be moved in a raster pattern across portions of the object 1010, such as groups in columns and rows of the object 1010. Alternatively, the excitation light may illuminate one or more entire regions of the object 1010 at one time and serially stop through the regions in a "step and shoot" scanning pattern. Line scanning can also be used as described, for example, in U.S. Pat. No. 7,329,860. The object 1010 produces the light signals 1040, which may include light emissions generated in response to illumination of a label in the object 1010 and/or light that has been reflected or refracted by an optical substrate of the object 1010. Alternatively, the light signals 1040 may be generated, without illumination, based entirely on emission properties of a material within the object 1010 (e.g., a radioactive or chemiluminescent component in the object).

The object and focus detectors 1042 and 1044 may be, for example photodiodes or cameras. In some embodiments herein, the detectors 1042 and 1044 may comprise a camera that has a 1 mega pixel CCD-based optical imaging system such as a 1002×1004 CCD camera with 8 gm pixels, which at 20× magnification can optionally image an area of 0.4×0.4 mm per tile using an excitation light that has a laser spot size of 0.5×0.5 mm (e.g., a square spot, or a circle of 0.5 mm diameter, or an elliptical spot, etc.). Cameras can optionally have more or less than 1 million pixels, for example a 4 mega pixel camera can be used. In many embodiments, it is desired that the readout rate of the camera should be as fast as possible, for example the transfer rate can be 10 MHz or higher, for example 20 or 30 MHz. More pixels generally mean that a larger area of surface, and therefore more sequencing reactions or other optically detectable events, can be imaged simultaneously for a single exposure. In particular embodiments, the CCD camera/TIRF lasers may collect about 6400 images to interrogate 1600 tiles (since images are optionally done in 4 different colors per cycle using combinations of filters, dichroics and detectors as described herein). For a 1 Mega pixel CCD, certain images optionally can contain between about 5,000 to 50,000 randomly spaced unique nucleic acid clusters (i.e., images upon the flow cell surface). At an imaging rate of 2 seconds per tile for the four colors, and a density of 25000 clusters per tile, the systems herein can optionally quantify about 45 million features per hour. At a faster imaging rate, and higher cluster density, the imaging rate can be improved. For example, a readout rate of a 20 MHz camera, and a resolved cluster every 20 pixels, the readout can be 1 million clusters per second. A detector can be configured for Time Delay Integration (TDI) for example in line scanning embodiments as described, for example, in U.S. Pat. No. 7,329,860. Other useful detectors include, but are not limited, to an optical quadrant photodiode detector, such as those having a 2×2 array of individual photodiode active areas fabricated on a single chip, examples of which are available from Pacific Silicon Sensor (Westlake Village, Calif.), or a position sensitive detector such as those having a monolithic PIN photodiode with a uniform resistance in one or two dimensions, examples of which are available from Hamamatsu Photonics, K.K., (Hamamatsu City, Japan).

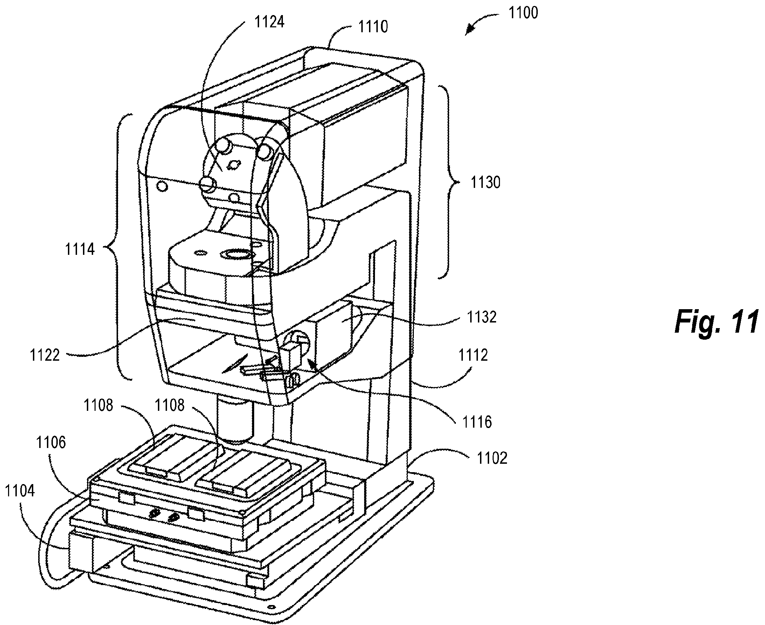

FIG. 11 is a perspective view of a sample imager 1100 formed in accordance with one embodiment. As shown, the sample imager 1100 includes an imager base 1102 that supports a stage 1104 having a sample holder 1106 thereon. The sample holder 1106 is configured to support one or more optical substrates 1108 during an imaging session. The optical substrates 1108 are illustrated as flow cells in FIG. 11. However, other samples may be used.

The sample imager 1100 also includes a housing 1110 (illustrated in phantom) and a strut 1112 that supports the housing 1110. The housing 1110 can enclose at least a portion of an optical assembly 1114 therein. The optical assembly 1114 may include a focus assembly 1116 and a sample-detecting assembly 1130. For example, the focus assembly 1116 may include an auto-focus line scan camera that receives reflected light beams for determining a degree-of-focus of the sampler imager 1100. The sample imager 1100 may also include a filter wheel 1122 and an alignment mirror 1124 that directs light toward a sample detector 1132, which is shown as a K4 camera in FIG. 11.

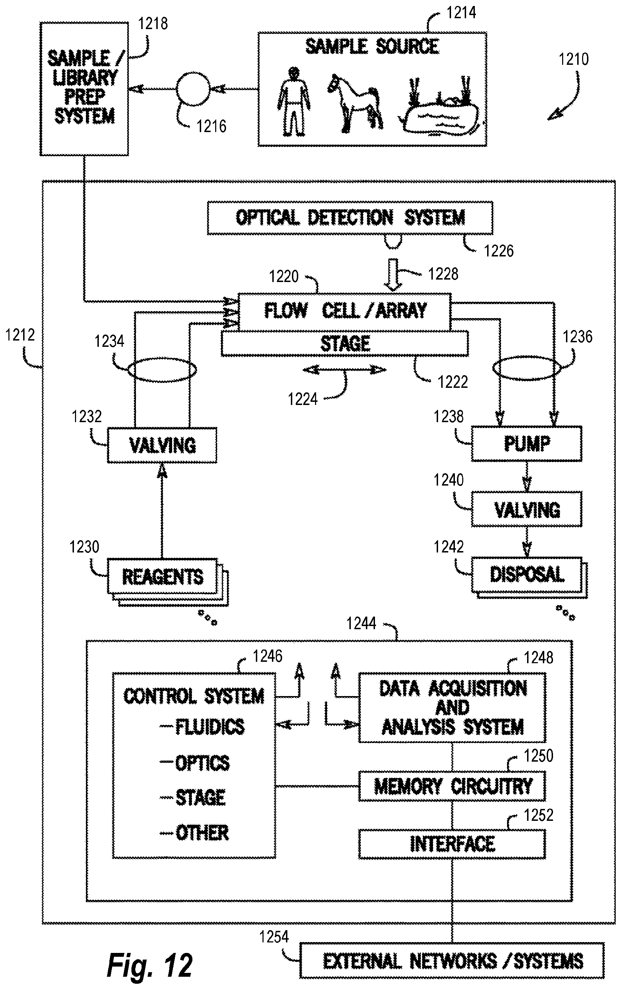

FIG. 12 illustrates an implementation of a sequencing system 1210 configured to process molecular samples that may be sequenced to determine their components, the component ordering, and generally the structure of the sample. The system includes an instrument 1212 that receives and processes a biological sample. A sample source 1214 provides the sample 1216 which in many cases will include a tissue sample. The sample source may include, for example, an individual or subject, such as a human, animal, microorganism, plant, or other donor (including environmental samples), or any other subject that includes organic molecules of interest, the sequence of which is to be determined. Of course, the system may be used with samples other than those taken from organisms, including synthesized molecules. In many cases, the molecules will include DNA, RNA, or other molecules having base pairs the sequence of which may define genes and variants having particular functions of ultimate interest.

The sample 1216 is introduced into a sample/library preparation system 1218. This system may isolate, break, and otherwise prepare the sample for analysis. The resulting library includes the molecules of interest in lengths that facilitate the sequencing operation. The resulting library is then provided to the instrument 1212 where the sequencing operation is performed. In practice, the library, which may sometimes be referred to as a template, is combined with reagents in an automated or semi-automated process, and then introduced to the flow cell prior to sequencing.

In the implementation illustrated in FIG. 12, the instrument includes a flow cell or array 1220 that receives the sample library. The flow cell includes one or more fluidic channels that allow for sequencing chemistry to occur, including attachment of the molecules of the library, and amplification at locations or sites that can be detected during the sequencing operation. For example, the flow cell/array 1220 may include sequencing templates immobilized on one or more surfaces at the locations or sites. A "flow cell" may include a patterned array, such as a microarray, a nanoarray, and so forth. In practice, the locations or sites may be disposed in a regular, repeating pattern, a complex non-repeating pattern, or in a random arrangement on one or more surfaces of a support. To enable the sequencing chemistry to occur, the flow cell also allows for introduction of substances, such as including various reagents, buffers, and other reaction media, that are used for reactions, flushing, and so forth. The substances flow through the flow cell and may contact the molecules of interest at the individual sites.

In the instrument the flow cell 1220 is mounted on a movable stage 1222 that, in this implementation, may be moved in one or more directions as indicated by reference numeral 1224. The flow cell 1220 may, for example, be provided in the form of a removable and replaceable cartridge that may interface with ports on the movable stage 1222 or other components of the system in order to allow reagents and other fluids to be delivered to or from the flow cell 1220. The stage is associated with an optical detection system 1226 that can direct radiation or light 1228 to the flow cell during sequencing. The optical detection system may employ various methods, such as fluorescence microscopy methods, for detection of the analytes disposed at the sites of the flow cell. By way of non-limiting example, the optical detection system 1226 may employ confocal line scanning to produce progressive pixilated image data that can be analyzed to locate individual sites in the flow cell and to determine the type of nucleotide that was most recently attached or bound to each site. Other imaging techniques may also suitably be employed, such as techniques in which one or more points of radiation are scanned along the sample or techniques employing "step and shoot" imaging approaches. The optical detection system 1226 and the stage 1222 may cooperate to maintain the flow cell and detection system in a static relationship while obtaining an area image, or, as noted, the flow cell may be scanned in any suitable mode (e.g., point scanning, line scanning, "step-and-shoot" scanning).

While many different technologies may be used for imaging, or more generally for detecting the molecules at the sites, presently contemplated implementations may make use of confocal optical imaging at wavelengths that cause excitation of fluorescent tags. The tags, excited by virtue of their absorption spectrum, return fluorescent signals by virtue of their emission spectrum. The optical detection system 1226 is configured to capture such signals, to process pixelated image data at a resolution that allows for analysis of the signal-emitting sites, and to process and store the resulting image data (or data derived from it).

In a sequencing operation, cyclic operations or processes are implemented in an automated or semi-automated fashion in which reactions are promoted, such as with single nucleotides or with oligonucleotides, followed by flushing, imaging and de-blocking in preparation for a subsequent cycle. The sample library, prepared for sequencing and immobilized on the flow cell, may undergo a number of such cycles before all useful information is extracted from the library. The optical detection system 1226 may generate image data from scans of the flow cell (and its sites) during each cycle of the sequencing operation by use of electronic detection circuits (e.g., cameras or imaging electronic circuits or chips). The resulting image data may then be analyzed to locate individual sites in the image data, and to analyze and characterize the molecules present at the sites, such as by reference to a specific color or wavelength of light (a characteristic emission spectrum of a particular fluorescent tag) that was detected at a specific location, as indicated by a group or cluster of pixels in the image data at the location. In a DNA or RNA sequencing application, for example, the four common nucleotides may be represented by distinguishable fluorescence emission spectra (wavelengths or wavelength ranges of light). Each emission spectrum, then, may be assigned a value corresponding to that nucleotide. Based upon this analysis, and tracking the cyclical values determined for each site, individual nucleotides and their orders may be determined for each site. These sequences may then be further processed to assemble longer segments including genes, chromosomes, and so forth. As used in this disclosure the terms "automated" and "semi-automated" mean that the operations are performed by system programming or configuration with little or no human interaction once the operations are initiated, or once processes including the operations are initiated.

In the illustrated implementation, reagents 1230 are drawn or aspirated into the flow cell through valving 1232. The valving may access the reagents from recipients or vessels in which they are stored, such as through pipettes or sippers (not shown in FIG. 12). The valving 1232 may allow for selection of the reagents based upon a prescribed sequence of operations performed. The valving may further receive commands for directing the reagents through flow paths 1234 into the flow cell 1220. Exit or effluent flow paths 1236 direct the used reagents from the flow cell. In the illustrated implementation, a pump 1238 serves to move the reagents through the system. The pump may also serve other useful functions, such as measuring reagents or other fluids through the system, aspirating air or other fluids, and so forth. Additional valving 1240 downstream of pump 1238 allows for appropriately directing the used reagent to disposal vessels or recipients 1242.

The instrument further includes a range of circuitry that aids in commanding the operation of the various system components, monitoring their operation by feedback from sensors, collecting image data, and at least partially processing the image data. In the implementation illustrated in FIG. 12, a control/supervisory system 1244 includes a control system 1246 and a data acquisition and analysis system 1248. Both systems will include one or more processors (e.g., digital processing circuits, such as microprocessors, multi-core processors, FPGA's, or any other suitable processing circuitry) and associated memory circuitry 1250 (e.g., solid state memory devices, dynamic memory devices, on and/or off-board memory devices, and so forth) that may store machine-executable instructions for controlling, for example, one or more computers, processors, or other similar logical devices to provide certain functionality. Application-specific or general purpose computers may at least partially make up the control system and the data acquisition and analysis system. The control system may include, for example, circuitry configured (e.g., programmed) to process commands for fluidics, optics, stage control, and any other useful functions of the instrument. The data acquisition and analysis system 1248 interfaces with the optical detection system to command movement of the optical detection system or the stage, or both, the emission of light for cyclic detection, receiving and processing of returned signals, and so forth. The instrument may also include various interfaces as indicated at reference 1252, such as an operator interface that permits control and monitoring of the instrument, transfer of samples, launching of automated or semi-automated sequencing operations, generation of reports, and so forth. Finally, in the implementation of FIG. 12, external networks or systems 1254 may be coupled to and cooperate with the instrument, for example, for analysis, control, monitoring, servicing, and other operations.

It may be noted that while a single flow cell and fluidics path, and a single optical detection system 1226 are illustrated in FIG. 12, in some instruments more than one flow cell and fluidics path may be accommodated. For example, in a presently contemplated implementation, two such arrangements are provided to enhance sequencing and throughput. In practice, any number of flow cells and paths may be provided. These may make use of the same or different reagent receptacles, disposal receptacles, control systems, image analysis systems, and so forth. Where provided, the multiple fluidics systems may be individually controlled or controlled in a coordinated fashion.

## Claims

1. A computer-implemented method comprising:
performing a variant analysis utilizing a variant analysis model to generate nucleotide base calls for a sample nucleotide sequence;
performing a diagnostic analysis on the nucleotide base calls to detect a genetic condition for the sample nucleotide sequence by utilizing a field programmable gate array (FPGA), locally housed on a shared network server with a container orchestration engine and the variant analysis model, to execute processes for diagnostic workflow containers in series as scheduled by the container orchestration engine with access to the nucleotide base calls within the shared network server, wherein diagnostic workflow containers make up an external sequencing diagnostic workflow from an external server without access to the nucleotide base calls within the shared network server;
isolating one or more workflow containers associated with respective functionalities of the external sequencing diagnostic workflow using targeted security permissions to prevent exposure of the nucleotide base calls to the external server; and
executing the external sequencing diagnostic workflow by utilizing the container orchestration engine to allocate computing resources of the FPGA for processing the one or more workflow containers scheduled in series.

2. The computer-implemented method of claim 1, wherein isolating the one or more workflow containers comprises controlling access to different workflow data sources for the one or more workflow containers to prevent access to sequencing data by the external sequencing diagnostic workflow.

3. The computer-implemented method of claim 1 or 2, further comprising receiving an indication of a label defining a version of the variant analysis model and a memory allocation to utilize for executing the external sequencing diagnostic workflow.

4. The computer-implemented method of any one of claims 1-3, further comprising:
designating a plurality of workflow data sources storing different types of workflow data; and
activating, for a workflow container from among the one or more workflow containers, access to a first workflow data source of the plurality of workflow data sources while preventing access to other workflow data sources of the plurality of workflow data sources.

5. The computer-implemented method of any one of claims 1-4, further comprising mounting a plurality of workflow data sources as read only for the one or more workflow containers.

6. The computer-implemented method of any one of claims 1-5, further comprising preventing the external sequencing diagnostic workflow from accessing sequencing data associated with the nucleotide base calls by applying read-only permissions to a workflow container that accesses the sequencing data and that is utilized by the external sequencing diagnostic workflow.

7. The computer-implemented method of any one of claims 1-6, further comprising executing the external sequencing diagnostic workflow during a variant analysis by the variant analysis model.

8. The computer-implemented method of any one of claims 1-7, wherein executing the external sequencing diagnostic workflow comprises implementing the external sequencing diagnostic workflow identified from an external application hosted on the external server separate from the shared network server of the variant analysis model and the container orchestration engine.

9. The computer-implemented method of any one of claims 1-8, further comprising:
determining a diagnostic execution mode corresponding to a standardized genetic diagnostic protocol; and
granting, for a client device, access only to diagnostic applications compatible with the diagnostic execution mode.

10. The computer-implemented method of any one of claims 1-9, further comprising preventing the external sequencing diagnostic workflow from accessing sequencing data associated with the nucleotide base calls by applying read-only permissions to a workflow container that accesses the sequencing data and that is utilized by the external sequencing diagnostic workflow.

11. The computer-implemented method of any one of claims 1-10, further comprising selectively preventing the one or more workflow containers from accessing sequencing data of the sample nucleotide sequence while executing the external sequencing diagnostic workflow.

12. The computer-implemented method of any one of claims 1-11, wherein isolating the one or more workflow containers comprises utilizing the targeted security permissions to individually designate sequencing data access permissions for the one or more workflow containers.

13. The computer-implemented method of any one of claims 1-12, further comprising receiving the external sequencing diagnostic workflow generated by an external device operated by an external entity.

14. A system comprising at least one processor and a non-transitory computer readable medium comprising instructions that, when executed by the at least one processor, cause the system to perform a method according to any one of claims 1-13.

15. A non-transitory computer-readable medium storing instructions that, when executed by at least one processor, cause a computing device to perform a method according to any one of claims 1-13.

## Patentansprüche

1. Computerimplementiertes Verfahren, umfassend:
Durchführen einer Variantenanalyse unter Verwendung eines Variantenanalysemodells zum Generieren von Nukleotidbasenaufrufen für eine Nukleotidsequenzprobe;
Durchführen einer diagnostischen Analyse der Nukleotidbasenaufrufs, um eine genetische Erkrankung der Nukleotidsequenzprobe zu erkennen, indem ein feldprogrammierbares Gate-Array (FPGA) verwendet wird, welches lokal auf einem gemeinsam genutzten Netzwerkserver mit einer Container-Orchestrierungs-Engine und dem Variantenanalysemodell untergebracht ist, um Prozesse für diagnostische Workflow-Container wie von der Container-Orchestrierungs-Engine geplant mit Zugriff auf die Nukleotidbasenaufrufe innerhalb des gemeinsam genutzten Netzwerkservers nacheinander auszuführen, wobei diagnostische Workflow-Container einen externen Sequenzierungs-Diagnose-Workflow von einem externen Server ohne Zugriff auf die Nukleotidbasenaufrufe innerhalb des gemeinsam genutzten Netzwerkservers bilden;
Isolieren eines oder mehrerer Workflow-Container, welche mit jeweiligen Funktionen des externen Sequenzierungs-Diagnose-Workflows verknüpft sind, mithilfe gezielter Sicherheitsberechtigungen, um die Offenlegung der Nukleotidbasenaufrufe gegenüber dem externen Server zu verhindern; und
Ausführen des externen Sequenzierungs-Diagnose-Workflows durch Verwenden der Container-Orchestrierungs-Engine zum Zuweisen von Rechenressourcen des FPGA für die Verarbeitung des einen oder der mehreren nacheinander geplanten Workflow-Container.

2. Computerimplementiertes Verfahren nach Anspruch 1, wobei Isolieren des einen oder der mehreren Workflow-Container Steuern des Zugriffs auf unterschiedliche Workflow-Datenquellen für den einen oder die mehreren Workflow-Container umfasst, um den Zugriff auf Sequenzierungsdaten durch den externen Sequenzierungs-Diagnose-Workflow zu verhindern.

3. Computerimplementiertes Verfahren nach Anspruch 1 oder 2, welches weiter Empfangen einer Angabe eines Etiketts umfasst, welches eine Version des Variantenanalysemodells und eine Speicherzuweisung definiert, welche zum Ausführen des externen Sequenzierungs-Diagnose-Workflows verwendet werden sollen.

4. Computerimplementiertes Verfahren nach einem der Ansprüche 1-3, weiter umfassend: Festlegen einer Vielzahl von Workflow-Datenquellen, welche unterschiedliche Arten von Workflow-Daten speichern; und
Aktivieren des Zugriffs auf eine erste Workflow-Datenquelle der Vielzahl von Workflow-Datenquellen für einen Workflow-Container aus dem einen oder den mehreren Workflow-Containern, während Zugriff auf andere Workflow-Datenquellen der Vielzahl von Workflow-Datenquellen verhindert wird.

5. Computerimplementiertes Verfahren nach einem der Ansprüche 1-4, welches weiter Einbinden einer Vielzahl von Workflow-Datenquellen als schreibgeschützt für den einen oder die mehreren Workflow-Container umfasst.

6. Computerimplementiertes Verfahren nach einem der Ansprüche 1-5, welches weiter Verhindern des Zugriffs des externen Sequenzierungs-Diagnose-Workflows auf Sequenzierungsdaten umfasst, welche mit den Nukleotidbasenaufrufen verknüpft sind, indem auf einen Workflow-Container, welcher auf die Sequenzierungsdaten zugreift und von dem externen Sequenzierungs-Diagnose-Workflow verwendet wird, Nur-Lese-Berechtigungen angewendet werden.

7. Computerimplementiertes Verfahren nach einem der Ansprüche 1-6, welches weiter Ausführen des externen Sequenzierungs-Diagnose-Workflows während einer Variantenanalyse durch das Variantenanalysemodell umfasst.

8. Computerimplementiertes Verfahren nach einem der Ansprüche 1-7, wobei Ausführen des externen Sequenzierungs-Diagnose-Workflows Implementieren des externen Sequenzierungs-Diagnose-Workflows umfasst, welcher aus einer externen Anwendung identifiziert wurde, welche auf dem externen Server getrennt von dem gemeinsam genutzten Netzwerkserver des Variantenanalysemodells und der Container-Orchestrierungs-Engine gehostet wird.

9. Computerimplementiertes Verfahren nach einem der Ansprüche 1-8, weiter umfassend:
Bestimmen eines Diagnoseausführungsmodus, welcher einem standardisierten genetischen Diagnoseprotokoll entspricht; und
Gewähren von Zugriff nur auf Diagnoseanwendungen, welche mit dem Diagnoseausführungsmodus kompatibel sind, für eine Client-Vorrichtung.

10. Computerimplementiertes Verfahren nach einem der Ansprüche 1-9, welches weiter Verhindern des Zugriffs des externen Sequenzierungs-Diagnose-Workflows auf Sequenzierungsdaten umfasst, welche mit den Nukleotidbasenaufrufen verknüpft sind, indem auf einen Workflow-Container, welcher auf die Sequenzierungsdaten zugreift und von dem externen Sequenzierungs-Diagnose-Workflow verwendet wird, Nur-Lese-Berechtigungen angewendet werden.

11. Computerimplementiertes Verfahren nach einem der Ansprüche 1-10, welches weiter selektives Verhindern des Zugriffs des einen oder der mehreren Workflow-Container auf Sequenzierungsdaten der Nukleotidsequenzprobe während Ausführen des externen Sequenzierungs-Diagnose-Workflows umfasst.

12. Computerimplementiertes Verfahren nach einem der Ansprüche 1-11, wobei Isolieren des einen oder der mehreren Workflow-Container Verwenden der gezielten Sicherheitsberechtigungen umfasst, um Sequenzierungsdaten-Zugriffsberechtigungen für den einen oder die mehreren Workflow-Container einzeln festzulegen.

13. Computerimplementiertes Verfahren nach einem der Ansprüche 1-12, welches weiter Empfangen des externen Sequenzierungs-Diagnose-Workflows umfasst, welcher von einer externen Vorrichtung generiert wird, welche von einer externen Entität betrieben wird.

14. System, welches zumindest einen Prozessor und ein nichtflüchtiges computerlesbares Medium umfasst, welches Anweisungen umfasst, welche, wenn sie von dem zumindest einen Prozessor ausgeführt werden, das System dazu veranlassen, ein Verfahren nach einem der Ansprüche 1-13 durchzuführen.

15. Nichtflüchtiges computerlesbares Medium, welches Anweisungen speichert, welche, wenn sie von zumindest einem Prozessor ausgeführt werden, eine Rechenvorrichtung dazu veranlassen, ein Verfahren nach einem der Ansprüche 1-13 durchführen.

## Revendications

1. Procédé mis en œuvre par ordinateur, comprenant :
la réalisation d'une analyse de variantes à l'aide d'un modèle d'analyse de variantes pour générer des appels de base nucléotidique pour une séquence nucléotidique d'échantillon ;
la réalisation d'une analyse de diagnostic sur les appels de base nucléotidique pour détecter une condition génétique pour la séquence nucléotidique d'échantillon en utilisant une matrice prédiffusée programmable par l'utilisateur (FPGA), hébergée localement sur un serveur de réseau partagé avec un moteur d'orchestration de conteneurs et le modèle d'analyse de variantes, pour exécuter des processus pour les conteneurs de flux de travail de diagnostic en série tels que planifiés par le moteur d'orchestration de conteneurs avec accès aux appels de base nucléotidique dans le serveur de réseau partagé, dans lequel les conteneurs de flux de travail de diagnostic constituent un flux de travail de diagnostic de séquençage externe à partir d'un serveur externe sans accès aux appels de base nucléotidique dans le serveur de réseau partagé ;
l'isolement d'un ou de plusieurs conteneurs de flux de travail associés à des fonctionnalités respectives du flux de travail de diagnostic de séquençage externe à l'aide de permissions de sécurité ciblées pour empêcher une exposition des appels de base nucléotidique au serveur externe ; et
l'exécution du flux de travail de diagnostic de séquençage externe en utilisant le moteur d'orchestration de conteneurs pour attribuer des ressources informatiques de la FPGA au traitement des un ou plusieurs conteneurs de flux de travail planifiés en série.

2. Procédé mis en œuvre par ordinateur selon la revendication 1, dans lequel l'isolement des un ou plusieurs conteneurs de flux de travail comprend le contrôle de l'accès à différentes sources de données de flux de travail pour les un ou plusieurs conteneurs de flux de travail pour empêcher un accès à des données de séquençage par le flux de travail de diagnostic de séquençage externe.

3. Procédé mis en œuvre par ordinateur selon la revendication 1 ou 2, comprenant en outre la réception d'une indication d'une étiquette définissant une version du modèle d'analyse de variantes et une attribution de mémoire à utiliser pour exécuter le flux de travail de diagnostic de séquençage externe.

4. Procédé mis en œuvre par ordinateur selon l'une quelconque des revendications 1-3, comprenant en outre : la désignation d'une pluralité de sources de données de flux de travail stockant différents types de données de flux de travail ; et
l'activation, pour un conteneur de flux de travail parmi les un ou plusieurs conteneurs de flux de travail, d'un accès à une première source de données de flux de travail de la pluralité de sources de données de flux de travail tout en empêchant un accès à d'autres sources de données de flux de travail de la pluralité de sources de données de flux de travail.

5. Procédé mis en œuvre par ordinateur selon l'une quelconque des revendications 1-4, comprenant en outre le montage d'une pluralité de sources de données de flux de travail en lecture seule pour les un ou plusieurs conteneurs de flux de travail.

6. Procédé mis en œuvre par ordinateur selon l'une quelconque des revendications 1-5, comprenant en outre le fait d'empêcher le flux de travail de diagnostic de séquençage externe d'avoir accès à des données de séquençage associées aux appels de base nucléotidique en appliquant des permissions en lecture seule à un conteneur de flux de travail qui a accès aux données de séquençage et qui est utilisé par le flux de travail de diagnostic de séquençage externe.

7. Procédé mis en œuvre par ordinateur selon l'une quelconque des revendications 1-6, comprenant en outre l'exécution du flux de travail de diagnostic de séquençage externe pendant une analyse de variantes par le modèle d'analyse de variantes.

8. Procédé mis en œuvre par ordinateur selon l'une quelconque des revendications 1-7, dans lequel l'exécution du flux de travail de diagnostic de séquençage externe comprend la mise en œuvre du flux de travail de diagnostic de séquençage externe identifié à partir d'une application externe hébergée sur le serveur externe séparé du serveur de réseau partagé du modèle d'analyse de variantes et du moteur d'orchestration de conteneurs.

9. Procédé mis en œuvre par ordinateur selon l'une quelconque des revendications 1-8, comprenant en outre :
la détermination d'un mode d'exécution de diagnostic correspondant à un protocole de diagnostic génétique standardisé ; et
l'octroi, pour un dispositif client, d'un accès uniquement à des applications de diagnostic compatibles avec le mode d'exécution de diagnostic.

10. Procédé mis en œuvre par ordinateur selon l'une quelconque des revendications 1-9, comprenant en outre le fait d'empêcher le flux de travail de diagnostic de séquençage externe d'avoir accès à des données de séquençage associées aux appels de base nucléotidique en appliquant des permissions en lecture seule à un conteneur de flux de travail qui a accès aux données de séquençage et qui est utilisé par le flux de travail de diagnostic de séquençage externe.

11. Procédé mis en œuvre par ordinateur selon l'une quelconque des revendications 1-10, comprenant en outre le fait d'empêcher de manière sélective aux un ou plusieurs conteneurs de flux de travail d'avoir accès à des données de séquençage de la séquence nucléotidique d'échantillon pendant l'exécution du flux de travail de diagnostic de séquençage externe.

12. Procédé mis en œuvre par ordinateur selon l'une quelconque des revendications 1-11, dans lequel l'isolement des un ou plusieurs conteneurs de flux de travail comprend l'utilisation des permissions de sécurité ciblées pour désigner individuellement des permissions d'accès aux données de séquençage pour les un ou plusieurs conteneurs de flux de travail.

13. Procédé mis en œuvre par ordinateur selon l'une quelconque des revendications 1-12, comprenant en outre la réception du flux de travail de diagnostic de séquençage externe généré par un dispositif externe exploité par une entité externe.

14. Système comprenant au moins un processeur et un support non transitoire lisible par ordinateur comprenant des instructions qui, lorsqu'elles sont exécutées par le au moins un processeur, amènent le système à réaliser un procédé selon l'une quelconque des revendications 1-13.

15. Support non transitoire lisible par ordinateur stockant des instructions qui, lorsqu'elles sont exécutées par au moins un processeur, amènent un dispositif informatique à réaliser un procédé selon l'une quelconque des revendications 1-13.
